Europäisches Patentamt

European Patent Office

Office européen des brevets

(19)

(11) **EP 1 543 836 A1**

(12) # EUROPEAN PATENT APPLICATION

(43) Date of publication:
**22.06.2005 Bulletin 2005/25**

(51) Int Cl.⁷: **A61K 39/108**, C12N 1/21

(21) Application number: **03029082.9**

(22) Date of filing: **17.12.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Berna Biotech AG**
**3018 Bern (CH)**

(72) Inventors:
• **Favre, Didier, Dr.**
**CH-3186 Düdingen (CH)**

• **Viret, Jean Francois, Dr.**
**CH-3177 Laupen (CH)**
• **Guido, Dietrich, Dr.**
**CH-3317 Laupen (CH)**

(74) Representative: **Grünecker, Kinkeldey,**
**Stockmair & Schwanhäusser Anwaltssozietät**
**Maximilianstrasse 58**
**80538 München (DE)**

(54) **Recombinant Vibrio cholerae strain and vaccine comprising said strain**

(57)    The present invention relates to a recombinant *Vibrio cholerae* strain, a vaccine comprising said strain, the use of said strain, and methods for its production. The recombinant strain according to the invention is comprising DNA sequences coding for at least the ETEC pili CFA/I, CS3 and CS6, wherein the DNA sequences are operationally linked to one or more promoters. The strain of the invention may be used in a vaccine composition for immunization against ETEC and/or cholera. The present invention further relates to methods for enhancing the surface expression of ETEC pili in a *V. cholerae* strain.

EP 1 543 836 A1

**Description**

Gram-negative enteric pathogens are the cause of a variety of diseases presenting with a broad spectrum of symptoms ranging from mild watery diarrhea to severe life-threatening symptoms such as fever, bloody diarrhea, perforation or ulceration of the stomach or intestine, alone or in combination. Examples of such diseases include, but are not limited to, typhoid fever, shigellosis, cholera, infections with enterotoxigenic, enteropathogenic, and enterohemorrhagic *Escherichia coli,* and infections with *Helicobacter pylori* and *Campylobacter jejuni.*

The first step of the infectious process of enteric pathogens occurs at the mucosal surface within the digestive tract. Thus, interfering with this initial stage of infection prior to the onset of symptoms offers a particularly attractive approach for providing protection against enteric diseases. The most effective means by which to accomplish this would be to evoke a local protective immune response through the use of an orally administered vaccine (Walker, 1994). At present, two live oral attenuated vaccines against enteric disease have been licensed for human use, namely the Ty21 a strain of *Salmonella typhi* for the prevention of typhoid fever and the CVD103-HgR strain of *Vibrio cholerae* for the prevention of cholera (Germanier and Fürer, 1975; Levine et al., 1988).

An effective protection against the enterotoxigenic *Escherichia coli* (ETEC) would be of particular medical interest. ETEC is one of the major causes of traveler's diarrhea and diarrhea among children in the developing countries and accounts for more than 1 billion diarrhea episodes and at least one million deaths per year, primarily among children.

ETEC isolates that cause diarrhea have several virulence factors that play important roles in the disease process. They include two enterotoxins, heat-labile toxin (LT) and heat-stable toxin (ST), as well as bacterial surface adhesins called pili or fimbriae which allow the organism to colonize the intestinal tract. Either one of the toxins can cause diarrhea. Some clinical ETEC isolates have been shown to produce either LT or ST, while other isolates have both toxins.

The most practical approach for the prevention of the widespread morbidity and mortality caused by diarrheal disease due to intestinal contamination with ETEC strains of *E. coli* would be by means of vaccination. The following three different types of *E. coli* antigen have been shown to be effective as immunogens which provide protection against challenge with ETEC strains in experimental models.

(i) Immunization of mice with either the *E. coli* LT holotoxin or its B subunit elicits an antitoxin response which provides protection against active challenge with the toxin itself and viable bacterial strains which produce either LT alone or both LT and ST toxins (Klipstein et al., 1981). However, immunizing with the holotoxin is not conceivable because of its toxicity to humans. On the other hand, the B subunit provides good immunogenicity and is nontoxic. However, since many ETEC strains produce only the ST toxin, an LT-B vaccine would only provide protection against a sub-group of ETEC infections from which all ST strains would be excluded. In contrast a combination of pili antigen and LT-B (or alternatively the cholera toxin B subunit [CT-B] which is highly homologous to LT-B) would be appropriate to protect against about 85% of all ETEC infections since it would cover many ST-producing strains.

(ii) Immunization with O-antigens prevents diarrhea by eliciting a mucosal immune response which helps reducing bacterial growth within the small intestine; this, however, extends only to homologous O-serotypes and not to heterologous O-serotypes. Since 170 antigenically dissimilar O-serotypes of *E. coli* including at least 78 for ETEC are known, immunization with O-antigens would require too many different vaccine strains to be effective (Wolf, 1997).

(iii) Immunization with fimbrial antigen (also known as pili or adhesins) responsible for adherence and colonization of the bacteria on the surface of the intestinal mucosa also provides protection. This protection, however, does not extend to ETEC strains possessing antigenically different fimbrial antigens (Morgan et al., 1978).

In addition to the above-mentioned *E. coli* immunogenic antigens, there is evidence that the B subunit of cholera toxin (CT-B) from *Vibrio cholerae* is also protective against ETEC strains expressing LT. This phenomenon may be explained by the similarity of CT-B and LT both in structure and in mode of action. Accordingly, cholera vaccines which include CT-B may be protective against ETEC diarrhea caused by ETEC isolates producing LT (Clemens et al., 1988). For example, Qadri et al. have recently shown that an oral vaccine containing a mixture of different ETEC pili antigens in combination with recombinantly produced cholera toxin B subunit (CT-B) is effective in children. In this phase II study carried out in Bangladesh, an oral vaccine comprising formalin-inactivated ETECs containing a total of six different surface antigens (CS 1-5 and CFA/I) were administered to children in combination with recombinant CT-B (Qadri et al, 2003). However, due to the use of killed cells, the level of immunogenicity afforded by this vaccine may not be optimal. As a consequence, two doses of the vaccine are necessary and the immunity may not be long-lasting. In contrast, live cells are thought to be more immunogenic, as they would more closely mimic a natural infection, also providing a longer-lasting immunity after only one dose (Levine and Kaper, 1993). Besides the fact that the inclusion of a purified toxin component raises the complexity and thus the cost of the vaccine, these considerations make it apparent that a live vaccine may be economically more viable.

An improvement with respect to this strategy would consist in delivering toxin-producing, pili-expressing live cells to the intestinal mucosa. Such a vaccine may be more efficiently sampled by the immune system due to its ability to penetrate the mucus layer overlaying the gut epithelium and adhere to the gut enterocytes. In turn, uptake by these and other specialized cells such as M cells with subsequent processing by antigen presenting cells would guarantee an optimal induction of a highly effective and specific mucosal immune response.

The induction of a local intestinal immune response may be the most efficient means by which to prevent infection with a number of enteric pathogens, including ETEC. A proven and effective method by which to accomplish this is through the use of live oral attenuated vaccine strains. Attenuated vaccine strains such as the above-mentioned *Salmonella typhi* Ty21a and *Vibrio cholerae* CVD103-HgR elicit an abortive infectious process thereby inducing an immune response closely resembling that effected by natural infection. In addition, *V. cholerae* CVD103-HgR has been shown to efficiently express a variety of foreign antigens in immunogenic form, among which the O-polysaccharide from *Shigella sonnei* and *V. cholerae* 0139, as well as shiga-like toxin Ib (Acheson et al 1993). Rabbits immunized with these constructs elicited significant antibodies directed against the heterologous antigen. Further, WO 94/19482 discloses a genetically engineered *V. cholerae* chromosome containing a heterologous DNA sequence encoding a bacterial antigen, wherein the DNA sequence is functionally linked to a naturally occurring *V. cholerae* promoter.

Both *Salmonella typhi* Ty21 a and *Vibrio cholerae* CVD103-HgR possess the distinct advantage of being approved as being extremely safe in humans (Dietrich et al, 2003). Safety, however, has been found to be the most difficult attribute to achieve in the development of live oral vaccine strains. Most often, candidate vaccine strains either induce a protective immune response but with an unacceptable rate of adverse reactions or are safe but non-protective (Levine and Hone, 1991).

In earlier studies, pili of ETEC have been expressed in the *Shigella flexneri* 2a and *Salmonella typhimurium* vaccine strains (Altboum et al. 2001; Wu et al. 1995). However, these approaches suffer from the fact that the type of immunity elicited by these strains is quite different than that elicited by ETEC. This is primarily due to the infection mechanism of these strains. Whereas Shigella and Salmonella are invasive organisms that cross the gut epithelium and infect cells of the immune system, ETECs are non-invasive. The efficacy of these vaccine strains as ETEC vaccines may therefore be limited, since the presentation of the ETEC antigen(s) to the immune system would not mimic that existing in nature. In this respect, expression from a vaccine strain with virulence characteristics similar to those of ETECs would be highly advantageous.

ETEC itself has also been recently used as carrier for homologous and heterologous pili. However, although immunogenic, these strains present various problems. The pilis can only be efficiently produced in solid CF medium at a temperature of about 37°C. Furthermore, ETEC cells are known to be releasedby infected people for extended periods of time. Finally, the risk exists of stably acquiring an ETEC plasmid carrying the wild type LT and/or ST toxin genes by conjugation, thereby producing a virulent strain out of a vaccine strain.

According to WO 03/022306, coli surface (CS) antigens may be expressed in ETEC strains. It is not clear, however, whether sufficient surface expression may be achieved.

The technical problem underlying the present invention is to develop a safe vaccine suitable to elicit an efficient immune response to a variety of ETEC isolates in human.

According to the present invention, this problem is solved by the provision of a recombinant *Vibrio cholerae* vaccine strain comprising DNA sequences coding for at least the ETEC pili CFA/I, CS3 and CS6, wherein the DNA sequences are operationally (functionally) linked to one or more promoters.

Since the virulence characteristics of wild-type *V. cholerae* are very similar to those of wild-type ETEC, the above-mentioned problems associated with the use of vaccine strains such as Shigella and Salmonella for the expression of ETEC adhesins are overcome by the recombinant *V. cholerae* strain according to the present invention. Both *V. cholerae* and ETEC colonize the small intestine without crossing the gut epithelium and secrete powerful toxins which mediate disease by causing net secretion into the intestinal lumen. The cholera toxin (CT) and the ETEC toxin (LT) share a homology of about 83% on the amino acid level and have an identical $AB_5$ structure. Most importantly, both pathogens elicit an immune response in a similar way. They are sampled by the immune system via the M cells of the Peyer's patches and elicit a local intestinal immune response through mucosal sIgA which can clear subsequent infection by immune exclusion.

The term "DNA sequence" refers to a nucleic acid sequence encoding the primary sequence of a protein. The term "ETEC pilus" as used herein refers to a polypeptide consisting essentially of, and having a similar immunological activity as the polypeptides listed in table 1. The immunological activity may be determined by parenteral immunization of mice and is considered as being "similar" if the subsequent detection of specific anti-pili antibodies in an enzyme-linked immunosorbent assay (ELISA) provides a significant titer. A significant ELISA titer is defined as being equal or superior to twice the reciprocal of the background ELISA titer.

The definition of "ETEC pilus" intended to encompass polypeptides comprising natural allelic variations, orthologs, artificially constructed variants, and derivatives of the pili listed in table 1. The term "ortholog" refers to a polypeptide from another species that corresponds to the human ETEC polypeptides as listed below. The term "variant" refers to a polypeptide comprising one or more amino acid substitution(s), deletion(s) and/or addition(s) as compared to the polypeptides as set forth in table 1. The term "derivative" refers to polypeptide allelic or artificially constructed variants and orthologs, as defined herein, that have been chemically modified.

Table1:

| ETEC pili types | | | |
|---|---|---|---|
| Pili type[a] | Genes | Genetic location and notes | Reference |
| CFA/I | *cfaA, B, C, E* | plasmid | Gaastra and Svennerholm, 1996 |
| CS1 (CFA/II) | *coo or csoA, B,* C, *D* | Plasmid | Marron et al, 1995 |
| CS2 (CFA/II) | *cotA, B, C, D* | Chromosome | Froehlich et al, 1995 |
| CS3 (CFA/II) | *cstA, B, C* | plasmid, cstB contains 5 overlapping ORF | Gaastra and Svennerholm, 1996 |
| CFA/III (CS8) | *cofA* | plasmid | Taniguchi et al, 1995 |
| CS4 (CFA/IV) | *csaA, B,* C, *E, D* | Plasmid | Altboum et al, 2003 |
| CS5 (CFA/IV) | *csfA, B, C, E, F, D* | Plasmid | Duthy TG et al, 1999 |
| CS6 (CFA/IV) | *cssA, B, C, D* | Plasmid | Gaastra and Svennerholm, 1996 |
| CS7 | | ? | Gaastra and Svennerholm, 1996 |
| CS10 (2230) | | Plasmid | Darfeuille-Michaud A. et al 1986 |
| CS11 (PCFO148) | | ? | Knutton S. et al, 1987 |
| CS12 (PCFO159) | | ? | Schmidt H et al., 1995 |
| CS13 (PCFO9) | | ? | Heuzenroeder MW et al, 1990 |
| CS14 (PCFO166) | $csuA_1, A_2, B, C, E$ | ? | Gaastra and Svennerholm, 1996 |
| CS15 (8786) | | Plasmid | Aubel et al, 1991 |
| CS17 | | ? | McConnell et al, 1990 |
| CS18 (PCFO20) | *fotA,B,C,D,E,F,G* | Chromosome (?) | Viboud et al, 1996 |
| CS19 | | ? | Grewal et al, 1997 |
| CS20 | | ? | Valvatne et al, 1996 |
| CS21 (Longus) | *IngA* | ? | Giron JA et al, 1997 |
| CS22 | *cseA* | ? | Pichel et al, 2000 |

[a] *In parentheses: previous denomination*

In humans, 21 different pili types have been described (see table 1 above), some of which occur together in the same organism. One pilus type, coli surface antigen 3 (CS3), is detected in 23% of ETEC isolates and occurs either alone or in combination with CS1 or CS2. CS6, detected in 21% of ETEC isolates, occurs either alone or in combination with CS4 or CS5. Colonization factor I (CFA/I) occurs alone in about one third of all ETEC isolates. These data are consistent with the idea that a potential vaccine comprising CFA/I, CS3 and CS6 may provide protection against a maximum of about 75% of all ETEC cases.

The *V. cholerae* strain according to the present invention may comprise further DNA sequence(s) encoding ETEC pili. In a preferred embodiment, the DNA sequence comprised in the *V. cholerae* strain according to the invention is selected from the group consisting of the sequences encoding the 21 pili described in table 1. Up to nine additional sequences coding for an ETEC pilus may be contained. Preferably, the strain of the invention comprises at least one further DNA sequence. More preferably, at least two or three further sequences are comprised. Optionally, pili from other pathogenic *E. coli* such as EaggEC (AAF/I pili), EHEC, EPEC (BFP, AF/R1), or veterinary ETEC (K88, K99, 987P), as well as from other bacteria may be included.

In a preferred embodiment, expression of the ETEC pili is driven by one or more ETEC promoter(s) (e.g. by the promoter naturally occuring in respective operon). Alternatively, promoter(s) form a source other than ETEC may be used (e.g. the lambda phage PL promoter [Schauder B et al., 1987] or the Ptrc promoter [Amann E et al., 1988]).

Genetically, the formation of CFA/I, CS3 and CS6 pili is encoded in operons comprising 4-7 genes each. Thus, in a preferred embodiment, the DNA sequence encoding the ETEC pilus comprises the entire operon.

In addition, the expression of many pili is up-regulated through the translational product of an additional gene which is encoded by a DNA sequence lying outside the respective operon. They are *cfaD* for CFA/I, *rns* for CS1 and CS2, and *csvR* and *csfR* for CS4. These regulatory genes show homology at the DNA level and were found to be functionally interchangeable (table 2).

Table 2:

| Regulators | | | |
|---|---|---|---|
| Genes | Pili regulated | Genetic location | Reference |
| CfaD | CFA/I | plasmid | Savelkoul et al, 1990 |
| CsvR | CS4, CS5 | plasmid | de Haan et al, 1991 |
| CsfR | CS4, CS5 | Cloned from CS6 strain E8775 | M.K.Wolf, pers.communic |
| Rns | CS1, CS2 | plasmid | Caron et al, 1989 |
| AggR | AAF/I | | Cassels and Wolf, 1995 |

In contrast to the expression of CFA/I, CS1, CS2 and CS4 in ETEC, which is enhanced by a regulator, expression of CS3 and CS6 is not enhanced in ETEC cells (Caron et al, 1990). That is, CS3 and CS6 expression obviously occurs independently from any regulation in their natural ETEC host. CFA/I expression is up-regulated by the *cfaD* gene product in ETEC. However, heterologous expression of CFA/I carried on a plasmid is known to occur without the need of a regulator in a foreign host such as a laboratory *E. coli* strain and *Salmonella typhimurium* (Wu et al. 1995).

Surprisingly in view of the above, the present authors found that expression of CFA/I and CS3 pili in a *V. cholerae* recipient strain was very poor even under the media and temperature conditions of growth that are normally known to lead to optimal pili synthesis. In contrast, the expression of CS6 pili was as good as in the ETEC wild-type strain B7A and thus considered satisfactory.

In order to improve the heterologous expression of CFA/I and CS3 in *V. cholerae*, expression of the CFA/I, CS3, and CS6 pili in the presence of the ETEC regulators was analyzed. For that purpose, the gene for either the csfR or the rns regulator was chromosomally integrated in strains carrying either the CFA/I or the CS3 locus. Expression of the ETEC regulator resulted in an increased expression of the structural pili proteins which translated into the surface formation of pili (which could be detected by various means including electron microscopy). Both CS3 and CFA/I expression was highly increased by either csfR or rns (see example 7 and 9). This was surprising since these operons are not supposed to be regulated inside ETEC or in a foreign host, respectively (see above). The expression of CS6 pilus was not increased by any of the regulators.

In a preferred embodiment of the present invention, expression of the ETEC pili is therefore enhanced by at least one ETEC regulator. The regulator may be selected from the group consisting of *csfR, rns, cfaD, csvR and aggR*. In one embodiment of the present invention the regulator is csfR. In another embodiment of the present invention the regulator is rns.

The term "regulator" refers to the translational product (polypeptide) encoded by any of the genes listed in table 2, as well as naturally occurring variations, orthologs, artificially constructed variants, and derivatives thereof.

In one embodiment of the present invention a recombinant *V. cholerae* strain is obtained by introducing the DNA sequences into a recipient strain selected from the group consisting of CVD103-HgR, CVD111, CVD112 and Peru-15. CVD103-HgR or an equivalent strain is particularly preferred. Like CVD103-HgR, equivalent strains show inactivation of the *ctxA* gene. Inactivation may preferably be achieved by deletion.

The term "recipient strain" refers to the strain which has been transformed, or is capable of being transformed with a DNA sequence and then of expressing a selected gene of interest. The progeny of said strain containing the selected DNA sequence is referred to as the recombinant strain. The term "parent strain" refers to the strain upstream of any given strain in the respective strain history.

CVD103-HgR is a live oral attenuated *V. cholerae* vaccine strain which originates from *V. cholerae* classical Inaba strain 569B and which has 94 % of the ctxA DNA sequence deleted but still expresses the immunogenic B subunit of CT (Ketley et al., 1993). As stressed above, expression of the ETEC adhesins from a live oral attenuated *V. cholerae* vaccine strain such as CVD103-HgR is advantageous, since the virulence characteristics of wild-type *V. cholerae* are very similar to those of wild-type ETEC. Other *V. cholerae* potential live vaccine strains which may be useful for the expression of ETEC pilis are CVD111 (Taylor et al, 1999) and Peru-15 (Kenner et al, 1995), both derived from El Tor O1 strains, and CVD112 (Tacket et al, 1995) and Bengal-15 (Coster et al, 1995), both derived from strains of the O139 serotype. Advantage of use of the above strains stem from the fact that i) *V. cholerae* El Tor is the only biotype currently isolated worldwide and ii) O139 is the only non-O1 serotype causing disease in humans.

In one embodiment of the invention, the DNA sequences coding for the pili are either present on a plasmid or stably integrated into the chromosome of the recipient strain.

The term "plasmid" is used to refer to a molecule capable of autonomous replication that is suitable for transformation of a recipient strain and contains DNA sequences that direct and/or control the expression of inserted heterologous DNA sequences. Various types of plasmids may be used such as low and high copy number plasmids, narrow and broad-host range plasmids, expression plasmids, and cosmids. High copy number plasmids are preferred, provided that they are not toxic to the cells. The latter seems to be case for the CS3 operon, for example. In these cases, low-copy number plasmids are preferred.

In general, heterologous gene expression is achieved by cloning of the heterologous gene(s) into plasmids which are replicated within the recipient in multiple copies thus leading to high expression of foreign gene product. However, synthetic plasmids are notably unstable, being rapidly lost from the cells when the latter are grown without selection pressure, giving rise to a cell population which does not express the heterologous antigen any longer. A further risk is that of plasmid(s) transfer to other bacterial species either in the environment or in the gut. Although some plasmids have been developed which address the first concern, the safety aspect remains a potential concern. Taken together, the expression of foreign antigens from plasmids is not appreciated by the regulatory authorities. Accordingly, the expression of foreign antigens is preferably achieved from corresponding genes integrated into the chromosome of the recipient strain, where they are stably maintained, while the risk of unwanted transfer to other organisms is excluded.

In order to circumvent both stability and safety concerns, the *cfa, cst,* and *css* loci encoding the CFA/I, CS3, and CS6 pili, respectively, preferentially are integrated into the chromosome of the *V. cholerae* CVD103-HgR recipient strain. The co-integration of 2-3 operons per one strain is preferred. In a preferred embodiment, the DNA sequence encoding the ETEC pilus is chromosomally integrated into an integration site which is non-essential for inducing a protective immune response by the strain, such as the *hlyA* and/or *hlyB* gene locus of *V. cholerae.* The *hlyB* locus is particularly preferred for integration.

Similarly, the DNA sequence encoding the regulator can either be present on a plasmid or stably integrated into the chromosome of the recipient strain. In a preferred embodiment, the DNA sequence encoding the ETEC regulator is chromosomally integrated into the *hlyA* and/or *hlyB* gene locus of *V. cholerae*. The *hlyB* locus is particularly preferred for integration.

In a further embodiment of the invention, multiple copies of the DNA sequence encoding the pilus and/or regulator are integrated in tandem into a single chromosomal integration site or independently integrated into individual integration sites. Preferably, 1 to 10 copies of the DNA sequence encoding the pilus and 1 to 10 copies of the DNA sequence encoding the regulator are inserted, more preferably 1-2 copies.

In vitro as well as in vivo, the expression of pili is temperature-dependent and occurs at around 37°C, usually less well at lower temperatures (Cassels and Wolf, 1995). It was therefore surprising that it proved to be possible to obtain the same level of expression after growth of the recombinant strains at 30 and 37°C. In a preferred embodiment of the invention, expression of the ETEC pili therefore occurs during growth in liquid medium at a temperature between 25°C and 42°C. Expression during growth at a temperature of 32-34°C is particularly preferred.

In one embodiment of the invention, the recombinant *V.cholerae* strain is characterized in that the strain has retained the characteristics of the recipient strain with respect to LPS and CT subunit B production, and cytotoxicity.

*V. cholerae* O1 Inaba lipopolysaccharide (LPS) may be identified by Western blotting with the specific monoclonal antibody VCO4 (Gustafsson and Holme, 1983). CTB production can be measured by the GM-1 ELISA assay as described (Svennerholm and Holmgren, 1978). A positive production of CTB is defined by a value of more than 2 μg/ml. Cytotoxicity may be determined by the Y1 adrenal assay as described (Sack and Sack, 1975) and is defined as a rounding of Y1 adrenal cells

In a preferred embodiment, the strain shows increased adherence to gut epithelial cells as compared to the recipient strain. The adherence may be determined by assessing the capacity of a cell expressing the respective polypeptide to adhere to gut cells. The adherence as defined in the present invention is considered to be "similar", if the normalized adherence index NAI is in the range of 0.9-1.1 if compared to that of CVD103-HgR (for details, see example 11); the adherence is considered to be "increased", if the normalized adherence index NAI is >1.

The capacity of the strain to adhere to gut cells may be determined as outlined in example 11 of the present description. Accordingly, an NAI above 1 indicates higher adherence than that of CDV103-HgR.

In another aspect, the present invention is related to a vaccine comprising the recombinant *V. cholerae* strain according to the invention, optionally in a mixture with a pharmaceutically acceptable excipient (e.g. lactose and/or ascorbic acid) and/or a buffer (e.g. sodium bicarbonate). In a preferred embodiment, the vaccine may further comprise other bacterial strains and/or toxins. Examples for toxins are given in the table below. A vaccine in which co-expression of 01 cholera LPS, the cholera toxin B-subunit and the ETEC pili is achieved may result in an enhanced protection rate and thus could resemble an alternative vaccine composition protective not only against ETEC, but also against cholera. By inclusion of an CT-B toxin, it may be possible to increase the protection rate to 85% of all ETEC isolates.

| TOXINS | | | |
|---|---|---|---|
| Genes | Toxins | | Reference |
| eltAB | LT-A, LT-B | heat-labile enterotoxin, plasmid | Sears and Kaper, 1996 |
| sta | ST-A, ST-B | heat-stable enterotoxin, plasmid | Sears and Kaper, 1996 |
| east1 | EAST | plasmid/chromosome | Yamamoto and Echeverria, 1996 |

In a further aspect, the present invention relates to the use of the recombinant *V. cholerae* strain according to the invention for preparing a pharmaceutical composition for immunization against ETEC and/or cholera.

Preferably, the pharmaceutical composition is for mucosal administration, most preferred is oral, nasal, and/ or rectal administration

In yet another aspect, the present invention is related to a method for producing the recombinant *V. cholerae* strain according to the invention, said method comprising the steps of

(a) introducing the DNA sequences coding for at least the ETEC pili CFA/I, CS3 and CS6 into a strain according to claim 8 and, optionally,
(b) further adding one or more of the DNA sequences coding for an ETEC regulator according to claim 5.

Further, the present invention also relates to a method for preparing a vaccine composition, comprising formulating the recombinant *V. cholerae* strain according to the invention in a mixture with a pharmaceutically acceptable excipient and/or buffer.

In another aspect, the present invention is related to methods for increasing the immunogenicity of a *V. cholerae* strain by increasing its adherence to the gut cells.

This may be achieved by introducing at least one DNA sequence encoding an ETEC pilus into the *V. cholerae* strain and culturing said strain under conditions which allow surface expression of the pili. Thus, ETEC pili are expressed on the surface of the *V. cholerae* cells in addition to homologous *V. cholerae* pili. In a preferred embodiment, at least one DNA sequence encoding a regulator gene is further introduced into the strain.

Certain *V. cholerae* strains, such as CVD103-HgR, are known to present an impaired adherence compared to their parent strains, e.g. CVD103 (Ketley et al., 1993). Furthermore, various studies suggest that some genetic modifications (Ketley et al., 1990), as well as the expression of foreign antigens in *V. cholerae* may reduce colonization with respect to the parent strain. In one study, for example, the addition of the eaeA gene of EHEC which codes for an outer membrane protein suppressed the immune response against the cloned subunit B of Shiga toxin. This effect may be due to lower growth and/or colonization by the eaeA-expressing strain (Butterton et al., 1997). In summary, the expression of a foreign antigen in *a V. cholerae* strain such as CVD103-HgR may adversely influence the adherence properties of said strain. This, in turn, may negatively influence the magnitude of the immune response elicited by the recombinant strain, since the induction of an efficacious immune response against *V. cholerae* is linked to the ability of the cells to adhere to the gut cells in order to i) express the CT toxin in vivo, and ii) to be sampled by the immune system at the Peyer's patches. It is therefore indispensable to guarantee the adherence of the recombinant strain. This may be achieved by expressing further adherence determinants in the recombinant strain, thereby increasing its adherence properties.

In the present invention, it was examined whether an increased adherence of CVD103-HgR could be achieved by expression of the ETEC pili in the *V. cholerae* strain. For that purpose, the adherence properties of the recombinant strain were compared to those of the recipient CVD103-HgR strain. It was found that, indeed, the adherence of the parent strain can be increased by this strategy (see example 11).

According to the invention, the surface expression of at least one ETEC pilus in a V. *cholerae* strain may further be enhanced by co-expression of a regulator gene which is stably integrated into the chromosome of the strain or present on a plasmid (see examples 7 and 9). For that purpose, at least one DNA sequence encoding a regulator gene is introduced into said strain and said strain is then cultured under conditions which allow expression of the pilus and the regulator, wherein the regulator gene is stably integrating into the chromosome of the strain or present on a plasmid. Preferentially, expression of CFA/I and/or CS3 may be enhanced using said method.

Alternatively, the surface expression of an ETEC pilus other than CS3 may be enhanced in a *V. cholerae* strain by

(a) introducing at least one DNA sequence coding for an ETEC pilus other than CS3 into a strain comprising a CS3 operon and at least one regulator gene stably integrated into the chromosome and
(b) culturing said strain under conditions which allow expression of the pilus and the regulator.

In a preferred embodiment of the invention, the recipient strain is CHCS3-R2, which is a CVD103-HgR strain containing an integrated CS3 pilus operon and an rns regulator within the *hlyB* gene. Using a co-integrated CS3 operon, CFA/I and/or CS6 preferentially are enhanced. In a further preferred embodiment, operons coding for the CFA/I or CS6 pilis are integrated within either the *hlyA, hlyB, lipA, or lipB* genes instead of the CS3 operon for enhancing the surface expression of an ETEC pilus other than CFA/I and CS6, respectively.

The following figures and examples shall illustrate the present invention in more detail. The examples given shall not be taken to limit the scope of the invention.

**Figures**

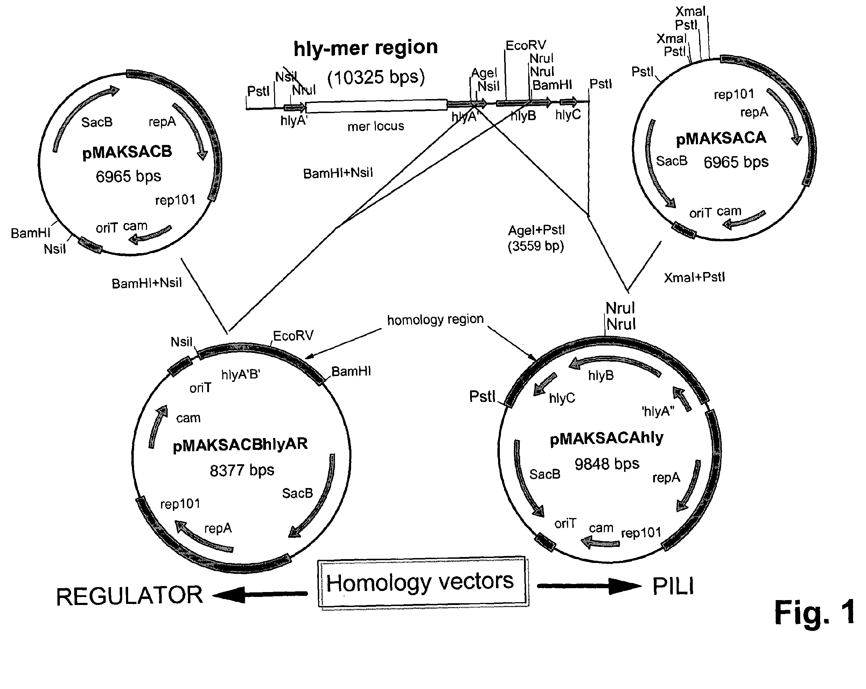

Fig.1: Construction of pili and regulator integration vectors. The arrows inside the plasmids represent the direction of transcription of the genes.

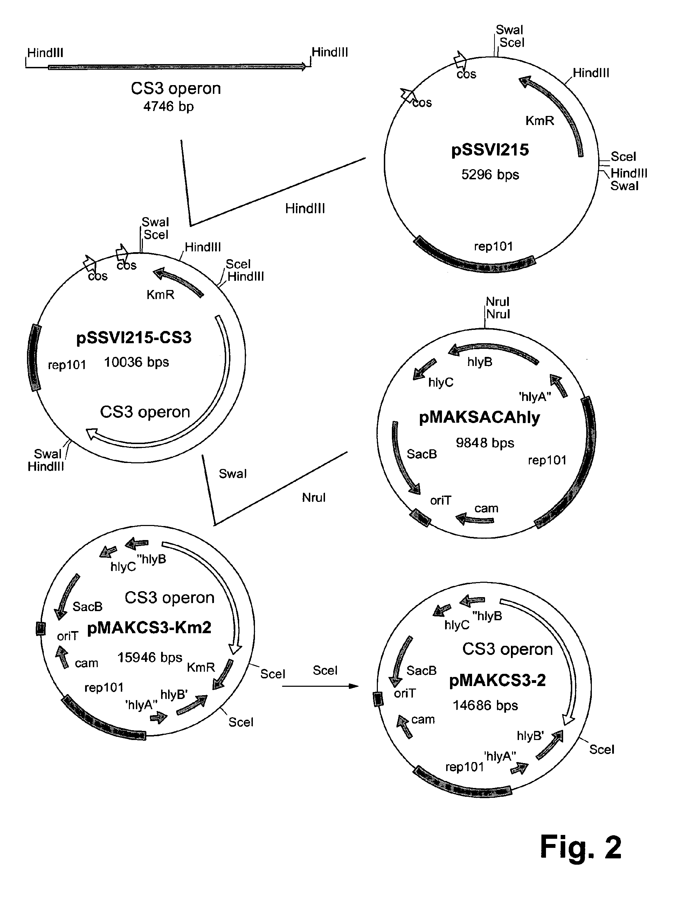

Fig.2: Construction of the CS3 operon integration plasmid pMAKCS3-2.

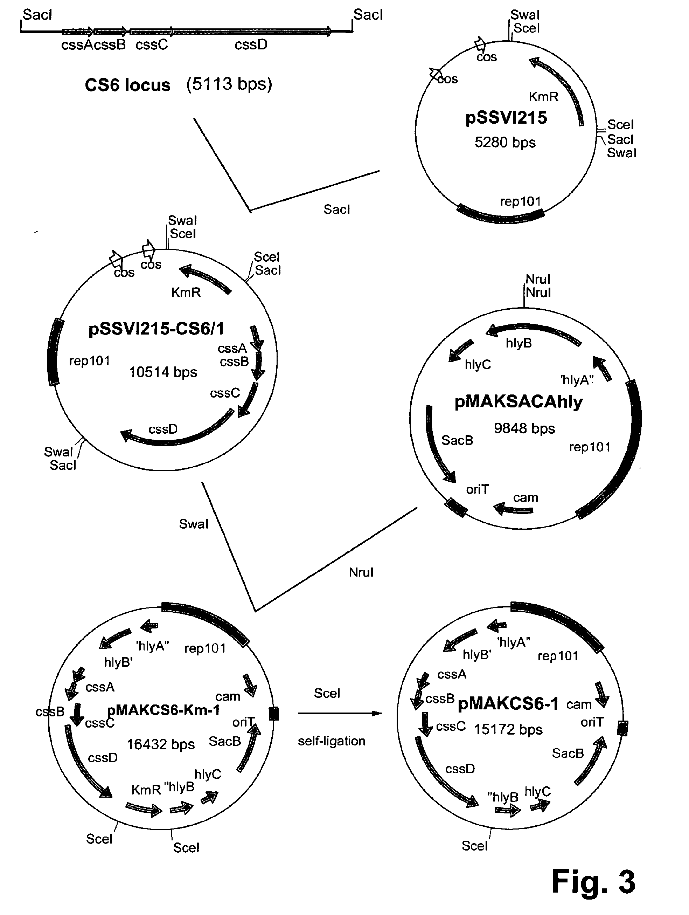

Fig.3: Construction of the CS6 operon integration plasmid pMAKCS6-1

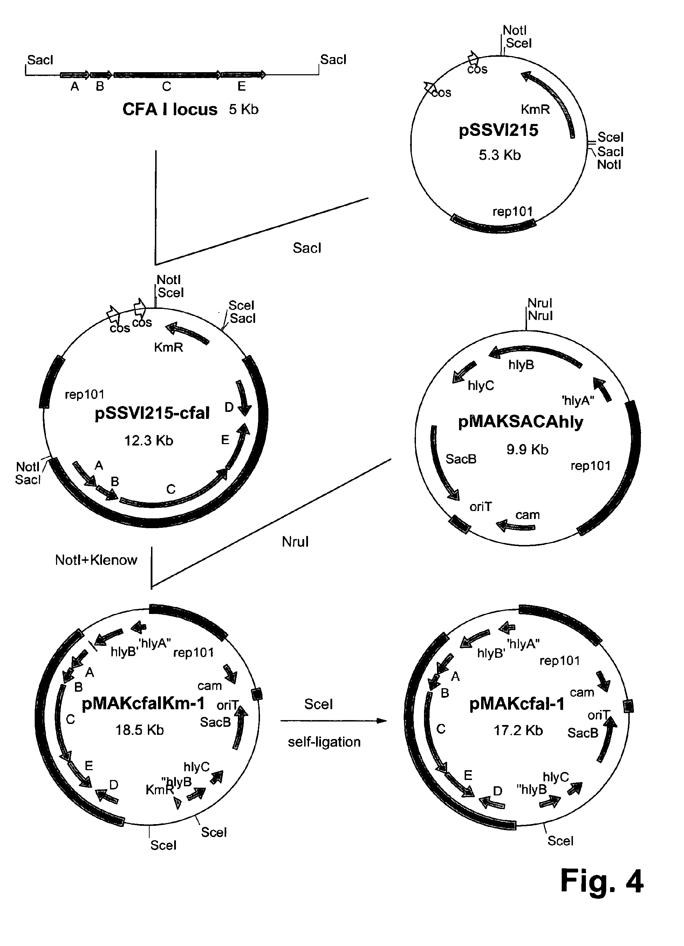

Fig.4: Construction of the CFA/I operon integration plasmid pMAKcfal-1

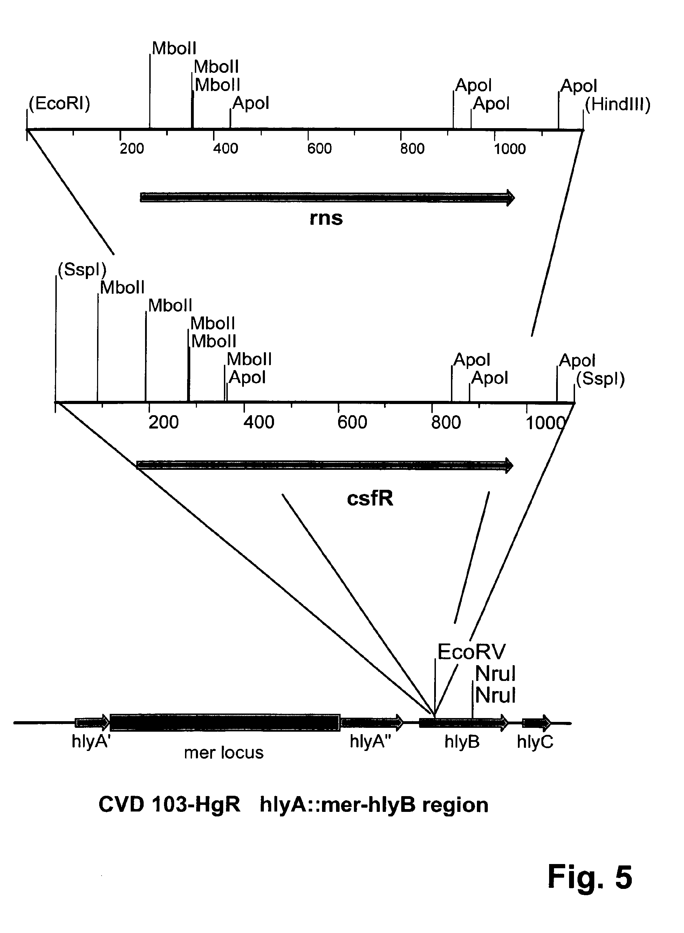

Fig 5: Integration of the regulator *csfR* or *rns* genes in the *hlyB* gene

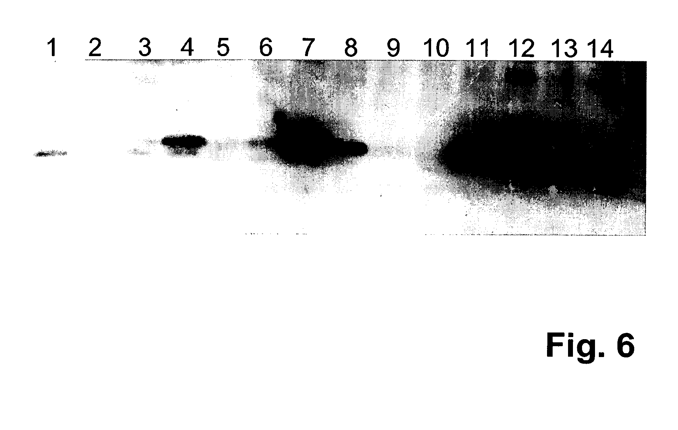

Fig.6: Western Blot of the expression of CS3 pilin in CVD103-HgR. The CS3 pilin was identified by use of a polyclonal CS3-specific rabbit antiserum. Lanes: 1. Wild-type ETEC DS7-3; 2. DH10B (pSSVI215); 3. DH10B (pSSVI215-CS3); 4. DH10B (pGB1); 5. CVD103-HgR (pSSVI215); 6. CVD103-HgR (pSSVI215-CS3); 7. CVD103-HgR (pSSVI215-CS3/pM392); 8. CVD103-HgR (pGB1); 9. CHCS3-2 colony #1; 10. CHCS3-2 colony #2; 11. CHCS3-2 #1 (pM392), 30°C.; 12. CHCS3-2 #1 (pM392), 37°C.; 13. CHCS3-2 #2 (pM392), 30°C.; 14. CHCS3-2 #2 (pM392), 37°C.

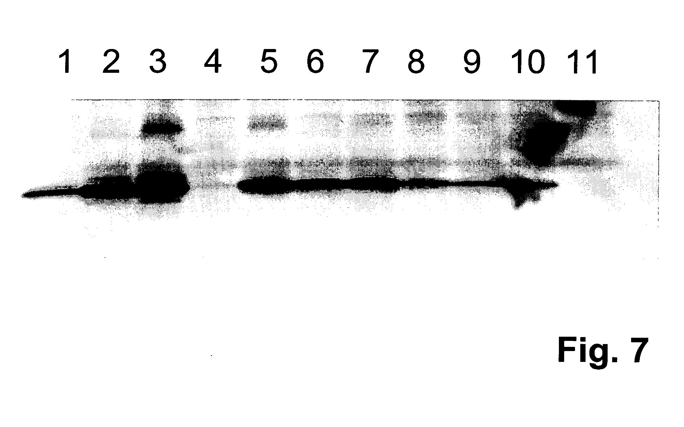

Fig.7: Western Blot analysis of the expression of CS6 pilin in CVD103-HgR. The CS6 pilin was identified by use of a polyclonal CS6-specific rabbit antiserum. Lanes: 1. ETEC B7A; 2. CVD103-HgR (pM295), 30°C; 3. CVD103-HgR (pM295), 37°C; 4. CVD103-HgR (pSSV1215); 5. CVD103-HgR (pSSV1215-CS6); 6. CVD103-HgR (pSSV1215-CS6/pM392), 30°C; 7. CVD103-HgR (pSSVI215-CS6/pM392), 37°C; 8. CHCS6-1; 9. CHCS6-1 (pM392); 10. CHCS6-2 (pM392); 11. CVD103-HgR (pSSVI215).

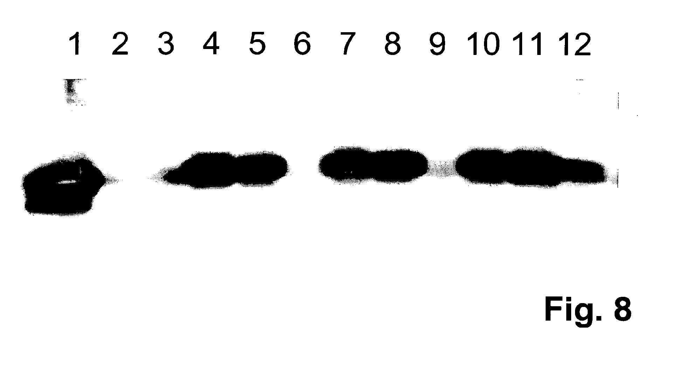

Fig.8: Western Blot analysis of the expression of CFA/I pilin in CVD103-HgR. The CFA/I pilin was identified by use of a polyclonal CFA/I-specific rabbit antiserum. Lanes: 1. Wild-type ETEC H10407; 2. CVD103-HgR (pSSVI215), 3. CVD103-HgR (pSSVI215-cfal); 4. CVD103-HgR (pSSVI215-cfal/pM392), 30°C; 5. CVD103-HgR (pSSVI215-cfal/pM392), 37°C; 6. CHcfal-1; 7. CHcfal-1 (pM392), 30°C; 8. CHcfal-1 (pM392), 37°C; 9. CHcfal-2; 10. CHcfal-1 (pM392), 30°C; 11. CHcfal-2 (pM392), 37°C; 12. CVD103-HgR (pK18cfal-a).

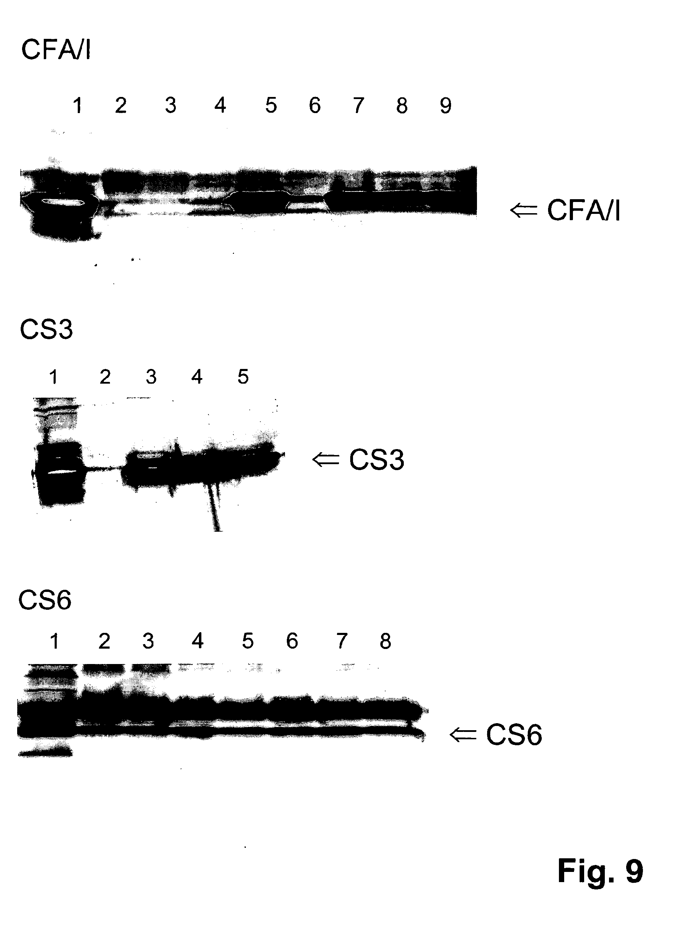

Fig.9: Western blot analysis of pilin expression in the presence of either the csfR or the rns regulator. Fig.4A (CFA/I), lanes: 1.ETEC H10407 2.CHcfal-1 3.CHcfal-2; 4.CHcfal-1 (pCLms); 5.CHcfal-2 (pCLrns); 6.CHcfal-1 (pEU2040); 7.CHcfal-2 (pEU2040); 8.CHcfal-1(pM392); 9.CHcfal-2 (pM392); Fig.4B (CS3), lanes: 1.DS7-3; 2.CHCS3-2; 3.CHCS3-2 (pCLms); 4.CHCS3-2 (pEU2040); 5.CHCS3-2 (pM392); Fig.4C (CS6), lanes: 1.ETEC B7A. 2.CHCS6-1; 3.CHCS6-2; 4.CHCS6-1 (pCLrns); 5.CHCS6-2 (pCLms); 6.CHCS6-1 (pEU2040) 7.CHCS6-2 (pEU2040) 8.CHCS6-1 (pM392).

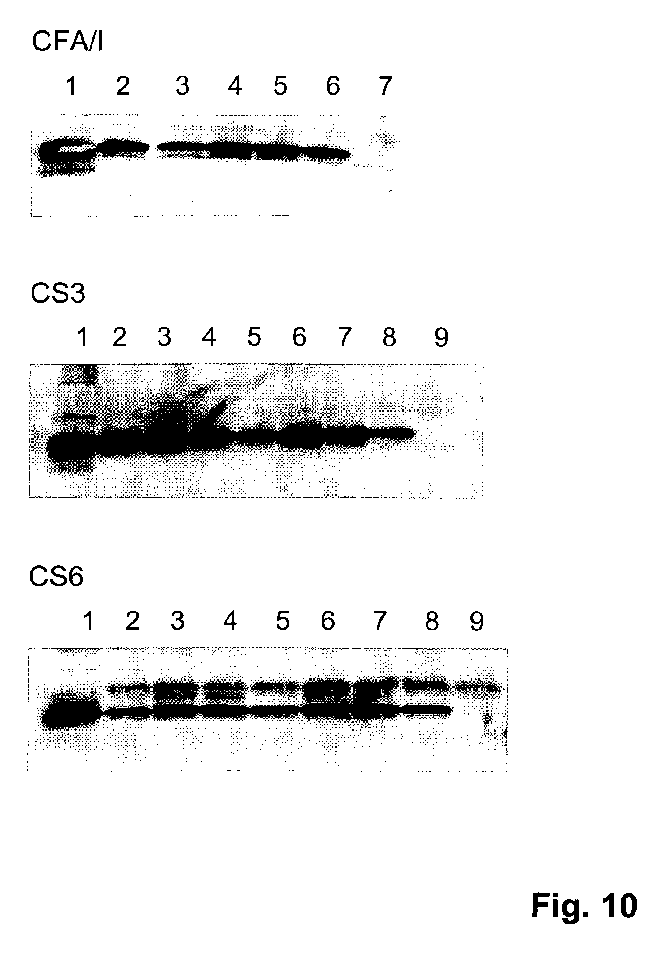

Fig.10: Western blot analysis of pili expression in recombinant CVD103-HgR/ETEC strains grown in liquid cultures at 37°C. Fig.10A(CFA/I), lanes: 1.ETEC H10407 grown on solid CF; 2.CHcfal-2 (pM392) grown on solid CF; 3.CHcfal-2 (pM392), 16 h in liquid CF; 4.CHcfal-2 (pM392) in liquid CF collected at $OD_{600}$= 0.5; 5.CHcfal-2 (pM392) in liquid CF collected at $OD_{600}$= 1.5; 6.CHcfal-2 (pM392) in liquid CF collected after 16 h incubation 7.CVD103-HgR grown on solid CF. Fig.10B (CS3), lanes: 1.ETEC DS7-3 grown on solid CF 2.CHCS3-2 (pM392) #1 grown on solid CF; 3.CHCS3-2 (pM392) #1 collected at $OD_{600}$ = 0.5; 4.CHCS3-2 #1 (pM392) CF collected at $OD_{600}$= 1.5: 5.CHCS3-2 (pM392) in liquid CF collected after 16 h incubation; 6.CHCS3-2 (pM392) #2 collected at $OD_{600}$ = 0.5; 7.CHCS3-2 #2(pM392) CF collected at $OD_{600}$= 1.5: 8.CHCS3-2 (pM392) #2 in liquid CF collected after 16 h incubation; 9.CVD103-HgR grown on solid CF. Fig.10C (CS6), lanes: 1.ETEC B7A grown on solid CF 2.CHCS6-1 grown on solid CF; 3.CHCS6-1 in liquid CF, pH 6.5 collected at $OD_{600}$ = 0.5; 4.CHCS6-1 grown in liquid CF, pH 6.5 collected at $OD_{600}$= 1.5: 5.CHCS6-1 in liquid CF, pH 6.5 collected after 16 h incubation; 6.CHCS6-1 in liquid CF, pH 7.3 collected at $OD_{600}$ = 0.5; 7.CHCS6-1 grown in liquid CF, pH 7.3 collected at $OD_{600}$= 1.5: 8.CHCS6-1 in liquid CF, pH 7.3 collected after 16 h incubation; 9.CVD103-HgR grown on solid CF.

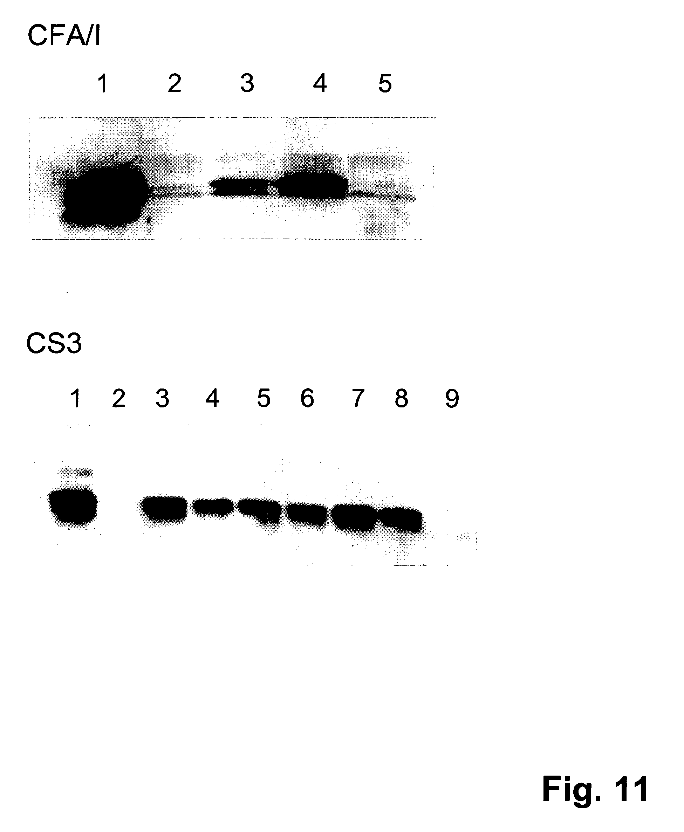

Fig. 11 Western blot analysis of pilin expression in recombinant CHCS3-2 containing an integrated *rns* regulator gene. Fig.11A (CFA/I), lanes: 1. H10407 ETEC; 2. CHcfal-1; 3. CHcfal-1 (pMAKSAChlyAR/rnsOri1); 4. CHcfal-1R1; 5. CVD103-HgR. Fig.11 B (CS3). lanes: 1. DS7-3, 37°C; 2. CHCS3-2, 37°C; 3. CHCS3-2 (pEU2040), 37°C; 4. CHCS3-2(pMAKSAChlyAR/rnsOri2), 30°C; 5. CHCS3-R2 #1, 37°C; 6. CHCS3-R2 #1, 30°C; 7. CHCS3-R2 #2; 37°C; 8. CHCS3-R2 #2, 30°C; 9. CVD103-HgR, 37°C.

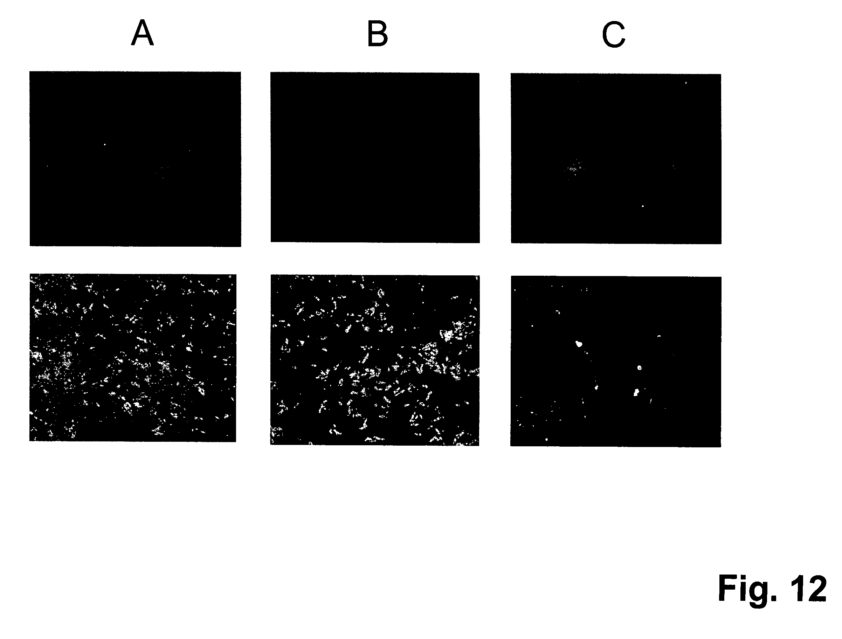

Fig.12: Immunofluorescence assay of the expression of ETEC pilis in CVD103-HgR. Panel A: top: CVD103-HgR; bottom: CHCFA-1 (pM392). Panel B: top: CVD103-HgR, bottom: CHCS3-2 (pM392); Panel C: top: CVD103-HgR, bottom: CHCS6-2.

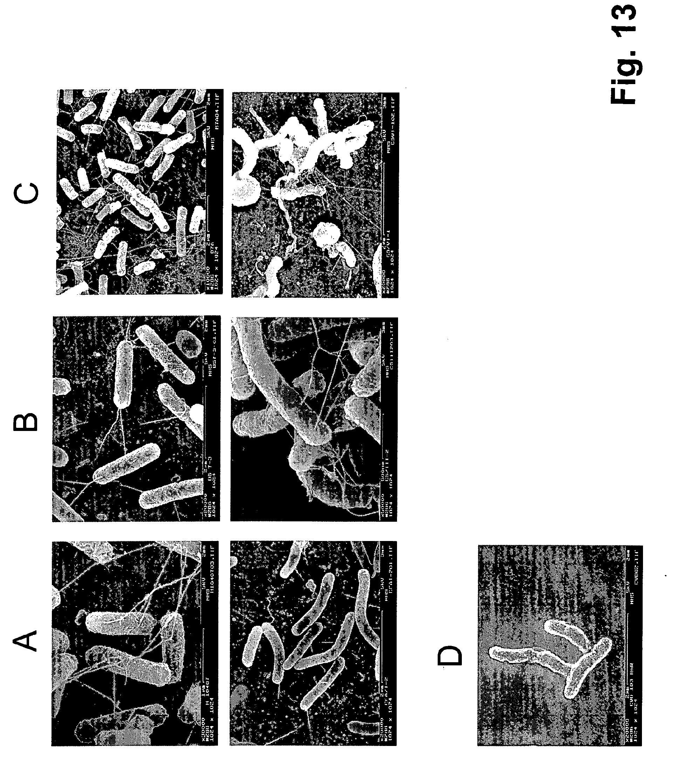

Fig.13: Ultrastructure of recombinant V. *cholerae*-ETEC strains by scanning electron microscopy. Panel A: top: ETEC H10407 (produces CFA/I pilis); bottom: CHCFA-1 (pM392); Panel B: top: ETEC DS7-3 (produces CS3 pilis), bottom: CHCS3-2 (pM392); Panel C: top: ETEC B7A (produces CS6 pilis), bottom: CHCS6-2; Panel D: CVD103-HgR.

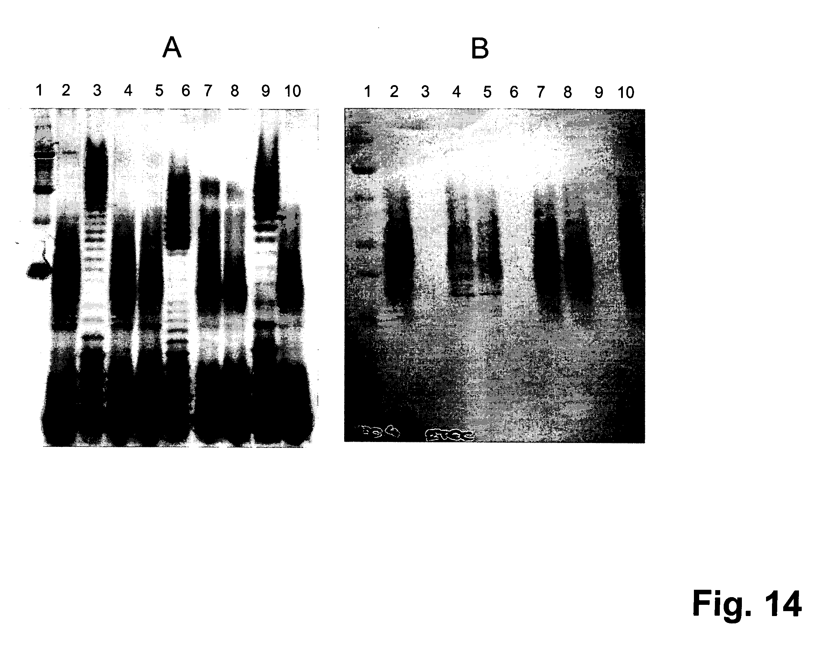

Fig. 14. LPS analysis of recombinant CVD103-HgR/ETEC strains. Panel A: Silver stained SDS-polyacrylamide gel. Panel B: Western blot probed with the *V.cholerae* O1 Inaba-specific Mab VCO4. Lanes: 1.Molecular weight standards. 2.CVD103-HgR 3.B7A. 4.CHCS6-1. 5.CHCS6-2. 6.DS7-3. 7.CHCS3-R2 #1 8.CHCS3-R2 #2 9.H10407. 10.CHcfa1-R1 # 2.

## Examples

### 1. Cultivation of strains

The bacterial strains and plasmids used in this study are listed in Table 3.

Table 3:

| Strains and plasmids | | |
|---|---|---|
| Strains | Genotype | Source or Reference |
| E.coli K12 | | |
| DH10B | *araD139, Δ(ara-leu7697), galU, galK, mcrA, Δ(mrr-hsdRMS-mcrBC), rpsL, deoR, Φ 80dlacZΔM15, endA1, nupG, recA1.* | |
| S17.1 | *thi-1 pro hsdR Tp$^r$ Sm$^r$ RP4-2 [ Tc::Mu (Km::Tn7)]* | Simon et al, |
| Enterotoxigenic E.coli | | |
| (ETEC) H10407 | *cfaABCE, LT, ST* | W.Gaastra |
| DS7-3 | *cstABC* | M.K. Wolf |
| B7A | *cssABCD* | M.K. Wolf |
| V. cholerae | | |
| CVD103-HgR | *ΔctxA hlyA::mer (Hg$^R$)* | Ketley et al, |
| CHcfal-1 | CVD103-HgR *hlyB::cfalABCE,* HlyB→cfal→ | 1993 this work |
| CHcfal-2 | CVD103-HgR *hlyB::cfalABCE,* HlyB→cfal← | this work |
| CHCS3-2 | CVD103-HgR *hlyB::cstABC* HlyB→cst← | this work |
| CHCS6-1 | CVD103-HgR *hlyB::cssABCD,* HlyB→css→ | this work |
| CHCS6-2 | CVD103-HgR *hlyB::cssABCD,* HlyB→css← | this work |
| Chcfa1-R1 | CHcfal-1 hlyB::*rns-1/cfalABCE* | this work |
| CHCS3-R2 | CHCS3-2 *hlyB::rns/cstABC* | this work |

Table 3:  (continued)

| Strains and plasmids | | |
| --- | --- | --- |
| Strains | Genotype | Source or Reference |
| plasmids | | |
| pK18 | Km[r] , 2.4 Kb, high copy number cloning plasmid | R.Pridmore |
| pCL1920 | Spc[r], 4.7 Kb low-copy number plasmid | C.Lerner |
| pMAKSACA | Cm', low copy number, sacB, rep101ts, suicide integration vector | Favre and Viret, 2000 |
| pMAKSACB | Cm[r], low copy number, sacB, rep101ts, suicide integration vector | Favre and Viret, 2000 |
| pSSVI215 | Km[r], low copy number, cos | Favre et al, |
| pM295 | source of *cssABCD* (CS6) locus | 1996 M.K. Wolf |
| pGB1 | source of *cstABC* (CS3) locus | D.A. Scott |
| pM392 | plasmid carrying the *csfR* regulator gene | M.K.Wolf |
| pM315 | source of the *csfR* regulator gene | M.K. Wolf |
| pEU2040 | source of the *rns* regulator gene | J.R.Scott |
| pJMK10 | Source of *hlyAB* locus | Ketley et al, |
| pCLRNS | pCL1920 with the 1.3 Kb HindIII-EcoRI *rns* fragment from pEU2040 | 1993 this work |
| pK18cfal-a | *cfal* locus cloned as a Psp1406I fragment in pK18 restricted by Smal | this work |
| pSSVI215-cfal | *cfal* locus cloned as a 7.3 Kb Sacl fragment in pSSVI215 cut by Sacl | this work |
| pSSVI215-CS3 | CS3 locus cloned as a 4746 bp HindIII fragment in HindIII-cut pSSVI215 | this work |
| pSSVI215-CS6 | CS6 locus cloned as a 5218 bp Sacl fragment in Sacl-cut pSSVI215 | this work |
| pMAKSACAhly | *hlyAB* locus as a 3559 bp PinAl-Pstl fragment from pJMK10 cloned in Xmal-Pstl-cut pMAKSACA | this work |
| pMAKcfal-Km1 | 8.6 Kb cfal-Km[r] Swal fragment from pSSVI215-cfal cloned in Nrul-cut pMAKSACAhly | this work |
| pMAKcfal-Km2 | as pMAKcfal-Km1 but cfal-Km[r] fragment in opposite orientation | this work |
| pMAKcfal-1 | pMAKcfal-Km1 restricted by I-Scel and self ligated to remove the Km[r] gene. Integration plasmid for the CFA/I locus. Transcription of CFA/I locus in the same direction as the hlyB gene | this work |
| pMAKcfa-2 | pMAKcfal-Km2 restricted by I-Scel and self ligated to remove the Km[r] gene. Integration plasmid for the CFA/I tocus. Transcription direction of CS6 locus opposite to that of the hlyB gene | this work |
| pMAKCS3-Km2 | 6 Kb Swal CS3-Km[r] fragment from pSSVI215-CS3 cloned in Nrul-cut pMAKSACAhlyA | this work |
| pMAKCS3-2 | pMAKCS3-Km2 restricted by I-Scel and self ligated to remove the Km[r] gene. Integration plasmid for the CS3 locus | this work |
| pMAKCS6-Km1 | 6 Kb Swal CS3-Km[r] fragment from pSSVI215-CS6 cloned in Nrul-cut pMAKSACAhlyA | this work |
| pMAKCS6-Km2 | as pMAKCS6-Km1 but CS6-Km[r] fragment in opposite orientation | this work |
| pMAKCS6-1 | pMAKCS6-Km1 restricted by I-Scel and self ligated to remove the Km[r] gene. Integration plasmid for the CS6 locus. Transcription of CS6 locus in the same direction as the hlyB gene | this work |
| pMAKCS6-2 | pMAKCS6-Km1 restricted by I-Scel and self ligated to remove the Km[r] gene. Integration plasmid for the CS6 locus. Transcription direction of CS6 locus opposite to that of the hlyB gene | this work |
| pMAKSACBhlyAR | 1653 bp BamHI - Nsil fragment from pJMK10 encompassing the 3'-end of the hlyA gene and the 5'-end of the hlyB gene cloned in BamHI-Nsil restricted pMAKSACB. | this work |

Table 3:   (continued)

| Strains and plasmids | | |
| --- | --- | --- |
| Strains | Genotype | Source or Reference |
| plasmids | | |
| pMAKcsfR-1 | a 1121 bp SspI fragment from pM392 into pMAKSACBhlyAR restricted by EcoRV. Transcription direction of csfR locus opposite to that of the hlyB gene | this work |
| pMAKcsfR-2 | csfR in opposite orientation with respect to that in pMAKcsfR-1 | this work |
| pMAKrns-1 | 1.2 Kb EcoRI-HindIII fragment from p into pMAKSACBhlyAR restricted by EcoRV | this work |
| pMAKrns-2 | rns in opposite orientation with respect to that in pMAKrns-1 | this work |

All *E. coli* K12 strains used for cloning, as well as enterotoxigenic *E. coli* were grown in LB (Luria broth) medium (Sambrook, Fritsch and Maniatis, 1989). For solid medium, 2% agar was added. Antibiotics were used at the following concentrations: chloramphenicol 17 μg/ml, kanamycin 50 μg/ml. For all strain construction purposes *V. cholerae* was grown on LBSOY consisting of 10g/L Soytone tryptone (Difco), 5 g/L Yeast extracts (Difco), and 10g/L NaCl, pH 7.3, or LBHySoy consisting of 10 g/L HySoy (Quest International, Bussum, Netherlands), 5 g/L Yeast extracts (Difco), and 10g/L NaCl, pH 7.2.

Pili expression was obtained by growing the recombinant *E. coli* or *V. cholerae* strains in CF medium consisting of 10.0 g/L Casamino acids, 1.5 g/L Yeast extracts, 50 mg/L $MgSO_4$ x $7H_2O$, and 5 mg/L $MnCl_2$ x $4H_2O$. The pH was adjusted to either 6.5 or 7.2 by NaOH. In cell adherence experiments, the bacteria were assayed on BHI plates (DIFCO).

Caco-2 cells for the adherence assay were seeded at $10^4$ cells/well and grown at 37°C under 5% $CO_2$ in MEM medium until almost confluent.

**2. Cloning of pili loci into integration vectors**

2.1. Preparation of the integration vector.

The integration vector was based on the suicide integration plasmid pMAKSACA (Favre and Viret, 2000) which was restricted with XmaI and PstI. The homology region derived from the plasmid pJMK10 (Ketley, et al, 1993), corresponding to a 3559 bp PinAI-PstI fragment encompassing the 3'-end of the hlyA gene, the entire hlyB gene and the 5'-end of the lipA gene, was cloned in the restricted vector to produce the vector pMAKSACAhly. pMAKSACAhly contains two NruI sites which are located 8 bp apart in the middle of the insert and within the hlyB gene. These NruI sites can be used for cloning of foreign genes to produce integration plasmids (figure 1).

2.2. Subcloning of the pili loci

2.2.1. CS3

The CS3 operon was excised from plasmid pGB1 as a 4.7 Kb HindIII fragment. This fragment was first cloned in the low-copy number vector pSSVI215 restricted by HindIII. The resulting plasmid was called pSSVI215-CS3 and contains the CS3 operon in tandem with a kanamycin-resistance gene (KmR) which serves as a positive marker for the subsequent subcloning. The 6 Kb CS3 operon-KmR gene fragment was further isolated by restricting pSSV1215-CS3 with SwaI and subcloned in pMAKSACAhly restricted by NruI. This produced the plasmid pMAKCS3-Km2, whereby the homology region is split between a left-hand segment of 1770 bp and a right-hand segment of 1781 bp. In order to remove the kanamycin-resistance gene, pMAKCS3-Km2 was excised by SceI and the large fragment was self-ligated. This resulted in the final 14.7 Kb integration plasmid called pMAKCS3-2 (figure 2).

2.2.2. CS6

The CS6 operon was excised from plasmid pM295 as a 4.5 Kb SacI fragment. This fragment was first cloned in the low-copy number vector pSSVI215 restricted by SacI. The resulting plasmid was called pSSVI215-CS6 and contains the CS6 operon in tandem with a kanamycin-resistance gene (KmR) which serves as a positive marker for the subsequent subcloning. The ca. 6 Kb CS6 operon-KmR gene fragment was further isolated from pSSVI215-CS6 by restriction with SwaI and subcloned in pMAKSACAhly restricted by NruI. This produced the plasmid pMAKCS6-Km-1 and pMAKCS6-Km-2, whereby the former plasmid contains the cssA, B, C, and D genes of the CS6 operon in the same orientation as the hlyB gene whereas the latter plasmid has the operon in opposite orientation to hlyB. The interruption of the hlyB gene by the CS6 locus produces a left-hand homologous segment of 1770 bp and a right-hand homologous segment of 1781 bp. In order to remove the kanamycin-resistance gene, pMAKCS6-Km-1 and pMAKCS6-Km-2, were excised by Scel and the large fragments were self-ligated. This resulted in the final 14.5 Kb integration plasmids called pMAKCS6-1 and pMAKCS6-2. Figure 3 shows the construction of the former plasmid.

### 2.2.3. CFA/I

The entire CFA/I operon was excised from plasmid pK18cfal-a as a 7.3 Kb Sacl fragment. This fragment was first cloned in the low-copy number vector pSSVI215 restricted by Sacl. The resulting plasmid was called pSSVI215-cfal and contains the CFA/I operon in tandem with a kanamycin-resistance gene (KmR) which serves as a positive marker for the subsequent subcloning. The ca. 8.6 Kb CFA/I operon-KmR gene fragment was further isolated from pSSVI215-cfal by restriction with Swal and subcloned in pMAKSACAhly restricted by Nrul. This produced the plasmids pMAKcfalKm-1 and pMAKcfalKm-2, whereby the former plasmid contains the genes of the CFA/I operon in the same orientation as the hlyB gene whereas the latter plasmid has the CFA/I operon in opposite orientation to hlyB. The interruption of the hlyB gene by the CFA/I locus produces a left-hand homologous segment of 1770 bp and a right-hand homologous segment of 1781 bp. In order to remove the kanamycin-resistance gene, pMAKcfalKm-1 and pMAKcfalKm-2 were excised by Scel and the large fragments were self-ligated. This resulted in the final 17.2 Kb integration plasmids called pMAKcfal-1 and pMAKcfal-2, respectively. Figure 4 shows the construction of the plasmids.

### 3. Construction of an integration vector for integration of the regulator genes

The integration vector was based on the suicide integration plasmid pMAKSACB (Favre and Viret, 2000) which was restricted with BamHI and Nsil, creating a 6724 bp vector. The homology region corresponded to a 1653 bp BamHI - Nsil fragment derived from pJMK10 (Ketley et al, 1993) encompassing the 3'-end of the hlyA gene and the 5'-end of the hlyB gene. The latter fragment was cloned into pMAKSACB to create pMAKSACBhlyAR. The latter vector contains a unique EcoRV cloning site within the homology region, providing 885 and 772 bp of homology arms, and located 245 bp downstream of the ATG start codon of the hlyB gene (figure 1).

### 4. Cloning of regulators into integration vectors

### 4.1. CsfR

The csfR regulator gene was cloned as a 1099 bp Sspl fragment into pMAKSACBhlyAR restricted by EcoRV. Two orientations of the gene within the vector were found by restriction analysis of the plasmids with Hpal and called pMAKcsfR-1 and pMAKcsfR-2. The former plasmid has csfR in the opposite orientation than the hlyB gene, whereas the latter plasmid has csfR in the same orientation (figure 5).

### 4.2. Rns

The Rns regulator gene was cloned as a ca., 1.2 Kb Klenowed EcoRI-HindIII fragment into pMAKSACBhlyAR restricted by EcoRV. Two orientations of the gene within the vector were found by restriction analysis of the plasmids with AlwNI and called pMAKrns-1 and pMAKrns-2. In plasmid pMAKrns-1, rns is transcribed in the reverse orientation with respect to hlyB gene transcription, whereas in pMAKrns-2, rns is transcribed in the same orientation as hlyB. ( figure 5)

### 5. Integration of pili and regulator loci into the chromosome of *V. cholerae*

### 5.1. Pili loci

In a first phase, the plasmids pMAKCS3-2, pMAKCS6-1, pMAKCS6-2, pMAKcfal-1, and pMAKcfal-2 were transferred in the *V. cholerae* live attenuated recipient vaccine strain CVD103-HgR by either transformation or conjugation via E. coli K12 S17.1 (Simon et al, 1983). In all subsequent steps, care was taken to grow cells on either the bovine-free medium called LBSOY consisting of 10g Soytone tryptone (Difco), 5 g Yeast extracts (Difco), and 10g NaCl or LBHySoy consisting of 10g HySoy (Quest), 5 g Yeast extracts (Difco), and 10g NaCl. For solid LBSOY or LBHySoy, 2% agar was added. Where needed, 17 µg/ml chloramphenicol was added to the plates (LBSOY Cm). Growth from a LBSOY Cm plate with CVD103-HgR (pMAKCS6-1 ) and (pMAKCS6-2) was inoculated in 1 ml PBS and homogenized.

Growth from CVD103-HgR (CHCS3-2), CVD103-HgR (pMAKcfal-1) and CVD103-HgR (pMAKcfal-2) was inoculated in 2 mls 0.9% NaCl and homogenized. 100 µl of 1 x, 10x, and 100x dilutions of these cell suspensions were plated out onto LBSOY Cm plates and incubated at 42°C for 16 hours. Individual colonies were then gathered and streaked out onto a 42°C pre-warmed LBSOY Cm plate and the plate was incubated at 42°C. Several of the resulting colonies were inoculated in 2 ml of liquid LBSOYS-N and incubated at 30°C during several hours. LBSOYS-N is LBSOY without NaCl and with 5% sucrose added. This medium allows the selection of integrants which have lost the vector sequences. The $OD_{600}$ of the cultures was measured and the cultures were serially diluted in order to have 100 to 10'000 CFU/plate. 100 µl dilutions were plated out onto LBSOYS-N plates, which were incubated at 30°C for 16 to 24 hours. Next, the plates containing 100 to 500 colonies were successively replica-plated onto LBSOYS-N Cm and LBSOYS-N. After overnight growth at 30°C, the colonies on both media were compared and 50 Cm-sensitive colonies were short-streaked onto new LBSOYS-N plates and grown at 30°C over night (O/N). These plates were subsequently blotted onto nylon filter and the presence of the CFA/I, CS3, and CS6 loci was tested by colony hybridization using specific DIG-labeled probes. A number of strains were found which have lost the vector sequences (Cm-sensitive) while conserving the pili loci. The new strains issued from pMAKCS3-2, pMAKCS6-1, pMAKCS6-2, pMAKcfal-1, and pMAKCFA/I-2 were named CHCS3-2, CHCS6-1, CHCS6-2, CHcfal-1, and CHcfal-2, respectively.

5.2. Regulators

Plasmids pMAKcsfR-1, pMAKcsfR-2, pMAKrns-1, and pMAKrns-2 were electroporated in CHcfal-1, CHcfal-2, and CHCS3 *V. cholerae* strains containing integrated pili loci. Each strain was grown onto LBSOY medium from glycerol stock cultures. The electroporation cultures were plated out onto LBSOY Cm medium and grown at 30°C. The integration procedure was then followed exactly as for the pili loci. Following integration, various integrants were screened for production of pilin. Two types of strains could thus be isolated, corresponding on the one hand to CHcfal-1 having integrated the ms gene from plasmid pMAKrns-1, and to CHCS3-2 having integrated the rns gene from plasmid pMAKrns-2 on the other hand. The strains were called CHcfa1-R1 and CHCS3-R2, respectively.

Many combinations of pili recombinant strains and regulator genes did not allow any pilin production, demonstrating that various factors may affect the expression of pilin from a combination of pili operon and regulator gene. Thus, the choice of such a combination is not a trivial one.

## 6. Western Blot assays of pili expression with or without regulator after growth on solid CF agar

The expression of pilin, the structural protein of the pili was studied by Western blotting. The strains to analyze were grown as lawns overnight on CF agar, pH 6.5 at the indicated temperature. Part of the lawn of bacteria was resuspended in 2 mls of 0.9% NaCl, the $OD_{600}$ was measured and the cell density was adjusted to an $OD_{600}$ of 3.0. One volume of the cell suspension was then mixed with 1 volume of SDS-PAGE sample buffer. 15 µl were subsequently run on a Criterion™ Precast Gel 4-20% (BioRad Laboratories, Glattbrugg, Switzerland). Following blotting against a nitrocellulose membrane, the membrane was blocked in 10% powdered milk (Rapilait) for 1 h at 37°C and incubated in the presence of a 10'000 dilution of pili-specific rabbit polyclonal antibodies. After three consecutive washes in PBS-T the membrane was incubated with a 10'000-fold dilution of a HP-conjugated goat anti-rabbit IgG (H + L). The membrane was again washed and the bands were detected by chemoluminescence after a 5 min contact with 1 ml ChemiGlow™ West Luminol / Enhancer Solution (AlphaInnotech, San Leandro, USA). The bands were detected using a video camera device (ChemiImager, AlphaInnotech, San Leandro,USA).

CS3

As shown in figure 6, the production of pilin from both the *E. coli* K12 and *V. cholerae* CVD103-HgR hosts appears to be dependent on the plasmid copy number. Thus in both cases expression is higher when the CS3 locus is expressed from plasmid pGB1 which is present at about 25 copies/cell (lanes 4 and 8) than from pSSVI215-CS3 which is present at about 3-5 copies/cell (lanes 3 and 6). Surprisingly, however, CS3 expression is vastly increased by co-expression of the csfR regulator, here carried on plasmid pM392. The action of the regulator is all the more spectacular that CS3 expression appears to be just as strong from the CS3 integrated locus as it is from the low-copy number clone pSSVI215-CS3 (compare lane 7 with lane 12 and 14). In contrast, the expression of the integrated locus in the absence of the regulator is extremely poor (lanes 9 and 10). Another surprising finding was that CS3 expression in the presence of the regulator was similar when the cells were grown at 30°C or 37°C. This is in contrast to the current understanding that in the wild type strain, pili expression is markedly better at 37°C than at 30°C.

CS6

In contrast to CS3, CS6 pilin appears to be synthesized in the absence of any regulator (figure 7, lanes 5 and 7). As a corollary, the expression of CS6 is dependent on plasmid copy-number. Thus, the highest amount of CS6 pilin stems from the high-copy-number plasmid pM295, followed by pSSVI215-CS6, and the integrated strain CHCS6-1 and CHCS6-2. However, it is noteworthy that even in the latter case, as much pilin is made as in the ETEC wild-type strain B7A. This underscores the fact that expression in CVD103-HgR is quite efficient, even from a single copy locus. As for CS3, it appears that, in CVD103-HgR the expression of CS6 is as good at 30°C than at 37°C.

CFA/I

The expression profile of CFA/I pilin resembles that of CS3. Figure 8 shows that, surprisingly, expression is highly dependent on the csfR regulator irrespective of whether the CFA/I locus is present on a plasmid (lanes 3 and 5) or chromosomally integrated (strains CHcfal-1 and CHcfal-2, compare lanes 6 and 8, as well as lanes 9 and 11). This appears to be in contrast with expression of CFA/I in foreign host which did not require a regulator (Wu et al, 1995). As in CS3, expression of CFA/I in the presence of the regulator is independent of the number of copies of the CFA/I locus since there is no difference in the amount of pilin made by the integrated strain or by the plasmid-encoded locus (compare lane 5 with lanes 8 or 11). Without a regulator, expression depends on the plasmid copy-number. Expression from the high-copy number pK18cfal-1 (lane 12) is higher than that from the low-copy number plasmid pSSVI215-cfal (lane 3). In contrast to the findings of Wu et al., expression of the locus in the absence of the regulator is poor (lanes 3, 6, and 9). A further surprising finding was that CFA/I expression in the presence of the regulator was similar when the cells were grown at 30°C or 37°C.

**7. Western Blot assays of pili expression using either the csfR or the rns regulator**

In a further aspect, we wished to check whether the use of any regulator was suitable to express CS3 and CFA/I pili. Accordingly, the rns regulator carried on plasmid pEU2040 (high copy number plasmid) or on pCLms (low-copy number plasmid) was electroporated in strains CHcfal-1, CHcfal-2, and CHCS3-2, and the expression of CFA/I and CS3, respectively, was analyzed by Western blot.

As the results depicted in figure 9 show, the expression from the rns or csfR regulators is very similar in magnitude. The only visible difference occurs in CHcfal-1. In this strain, the csfR regulator allows strong expression of the CFA/I pilin whereas the ms regulator does not. This shows that, in contrast to the current knowledge, the csfR and rns regulators are not completely interchangeable.

**8. Western Blot assays of pili expression after growth on liquid CF agar**

Current knowledge holds that the ETEC pili (and, presumably pilins) are best expressed on solid CF plates. We therefore checked if this holds true in the case of *V. cholerae*-expressed pilin. In addition, the effect on pilin expression of CF medium at either pH 6.5 or pH 7.3 was compared. Overnight LBHySoy cultures of the strains to test were either spread as lawns of cells on a CF or chloramphenicol-containing CF (CFCm) plate, pH 6.5, or diluted in 10 mls of CF or CFCm medium, pH 6.5 or 7.3. Liquid and solid cultures were grown at 37°C. From the CF/CFCm plates, cell suspensions in 0.9% NaCl were made to an $OD_{600}$ of 3.0. These suspensions served as reference for production of pilin from solid CF. The overnight CF/CFCm cultures were diluted to an $OD_{600}$ of 0.1 in 30 ml of fresh CF/CFCm medium at pH 6.5 or 7.3 and the cultures were agitated at 37°C. At $OD_{600}$ of 0.5, 1.5, as well as after overnight growth, samples of the cultures were withdrawn, pelleted and resuspended to an $OD_{600}$ of 3.0 in 0.9% NaCl. Finally, 100 μl of the cell suspensions at $OD_{600}$ =3.0 were mixed with 100 μl of sample buffer and 15 μl of each sample was run on a 4-20% gradient SDS-PAGE gel. After the run, the gel was blotted onto nitrocellulose membrane and the pilin proteins were detected by means of specific polyclonal antibodies.

The results are presented in figure 10. Surprisingly, pilin from any of the pili types, i.e., CFA/I, CS3, or CS6 was very well expressed in liquid CF. There is no significant difference in the amount of pilin made from bacteria grown on plates or in liquid medium. Whereas CS6 pilin is made in equivalent amounts at all growth phases, CS3 and CFA/I pilin seem to be made in larger amount in exponential phase than in stationary phase. The difference is however rather small. In addition, there is no difference between growth in CF medium pH 6.5 or 7.3. In conclusion, pilin is very well synthesized by *V. cholerae* strains carrying pili loci and regulators grown in liquid cultures.

**9. Expression of pilin from recombinant strains with an integrated regulator gene.**

The amount of pilin synthesized by strains containing the integrated rns regulator gene was tested by Western blotting. Figure 11, panel A (CFA/I) indicates excellent synthesis of CFA/I pilin by CHcfa1-R1 strain (lane 4) even compared to the CVD103-HgR carrying a plasmid-borne *rns* gene (compare lanes 3 and 4). Therefore, the integration of the ms gene leads to a very good expression of the CFA/I pilin. Likewise, panel B (CS3) shows that the expression of the CS3 pilin is just as strong from the CHCS3-R strains which possess the integrated rns gene (lanes 5-8), as it is from strains carrying the plasmid-borne *rns* gene (lanes 3-4).

**10. Co-expression of two pilis in the same recipient strain**

We investigated the possibility to co-express two different pilis in a single strain. We chose CHCS3-R2 as the recipient strain because of its excellent surface expression of the CS3 pili. In preliminary experiments, the plasmids pSSVI215-cfal or pSSVI215-CS6 were electroporated into CHCS3-R2. The expression of both the CS3 and CFA/I pilis in strain CHCS3-R2 (pSSVI215-cfal), as well as the expression of the CS3 and CS6 pilis in the strain CHCS3-R2 (pSSVI215-CS6) were studied by Western blotting, immunospots and immunofluorescence.

10.1. Western blot

A Western Blot was carried out as described above (see example 6). The results are shown in table 4.

10.2. Immunospots immunofluorescence

To determine the immunofluorescence, the method of Lang et al, 1990 was followed. Briefly, the strains indicated in table 4 were grown onto CF plates at 37°C. A cell suspension was then made in 0.9% NaCl at an $OD_{600}$ of 1.0-2.0. A loopful of the suspension was smeared on a glass slide and air-dried. The slides were then dipped in cold acetone (-20°C) for 10 min at 4°C and air-dried. The glass slides were washed 3 x in PBS, air dried, and 100 µl of a 50 times dilution of each of the specific antisera were applied to the corresponding dried cells and incubated at room temperature for 15 min. The slides were again washed 3x in PBS, air-dried and 100 µl of a 1:100 dilution of a FITC-labeled anti-rabbit IgG antibody was applied to the cells at room temperature for 15 min. The slides were again washed 3x by PBS and air-dried. Finally, 5 µl $H_2O$ and a drop of MOVIOL was added to each bacterial spot and the preparation was covered with a cover slip. The results were recorded on a Nikon Eclipse E800 fluorescence microscope.

For detection of immunospots, the strains indicated in table 4 were grown in CF medium, resuspended in 0.9% NaCl to an $OD_{600}$ of 3.0 and diluted to an $OD_{600}$ of 0.3. 5 µl of the undiluted and diluted cell suspensions were spotted on a nitrocellulose filter. The filter was blocked with 10% horse serum. Then the filter was incubated for 1 h with a 1:10'000 dilution of the specific polyclonal antibody at room temperature. After extensive washing the filter was further incubated for 30 min at room temperature with a 1:10'000 dilution of a horseradish peroxydase-conjugated anti-rabbit IgG antibody. After washing, the filter was either incubated with a chemolumiescent substrate or with a colorimetric substrate. In the former case, the development of chemoluminescence is detected with a video camera device (Chemilmager, Alphalnnotech, San Leandro CA, USA) or by colorimetric development of the signal.

Table 4:

| Co-expression of pilis with CHCS3-R2 as the recipient strain | | | | |
|---|---|---|---|---|
| Primary antibody | Strain | Western blot[a] | Immunospots[b] | Immunofluorescence[c] |
| | H10407 | - | + | + |
| | DS7-3 | + | ++ | +++ |
| | B7A | - | - | - |
| CS3 | CHCS3-R2 | +++ | ++ | ++ |
| | CHcfa1-R1 | - | - | - |
| | CHCS6-1 | - | - | - |
| | CHCS3-R2 (pSSVI215-cfal) | +++ | ++ | ++ |

[a] - no band, +: thin band, ++ medium band, +++ thick band

[b] - no visible spot, +: clear spot at $OD_{600}$ = 3.0 but weak at 0.3, ++: clear spots at both ODs.

[c] - no fluorescence, cells almost undetectable. +: weak fluorescence. ++: medium fluorescence, readily visible. +++: strong fluorescence.

Table 4:   (continued)

| Co-expression of pilis with CHCS3-R2 as the recipient strain | | | | |
|---|---|---|---|---|
| Primary antibody | Strain | Western blot[a] | Immunospots[b] | Immunofluorescence[c] |
| | CHCS3-R2 (pSSVI215-CS6) | +++ | ++ | ++ |
| | CVD103-HgR | - | - | - |
| | | | | |
| | H10407 | ++ | ++ | +++ |
| CFA/I | DS7-3 | - | - | - |
| | CHCS3-R2 | - | - | - |
| | CHcfa1-R1 | + | ++ | ++ |
| | CHCS3-R2 (pSSVI215-cfaI) | ++ | ++ | ++ |
| | CVD103-HgR | - | - | - |
| | | | | |
| | B7A | ++ | + | +++ |
| | DS7-3 | - | - | - |
| CS6 | CHCS3-R2 | - | + | - |
| | CHCS6-1 | + | + | - |
| | CHCS3-R2 (pSSVI215-CS6) | +++ | ++ | + |
| | CVD103-HgR | - | - | - |

[a] - no band, +: thin band, ++ medium band, +++ thick band

[b] - no visible spot, +: clear spot at $OD_{600}$ = 3.0 but weak at 0.3, ++: clear spots at both ODs.

[c] - no fluorescence, cells almost undetectable. +: weak fluorescence. ++: medium fluorescence, readily visible. +++: strong fluorescence.

Table 4 summarizes the results. Immunospots and immunofluorescence results correlate well with each other. In all CHCS3-R2 strains the CS3 pili is very well expressed. In the CHCS3-R2 (pSSVI215-cfaI) strain CFA/I pili is also very well expressed. Surprisingly, expression is even much higher than in the singly integrated strain CHcfa1-R1. Likewise, while the expression of the CS6 pili in CHCS6-1 is very weak, a reinforcement of expression was seen in the double expression strain CHCS3-R2 (pSSVI215-CS6). In conclusion, the expression of both CFA/I and CS6 pili surprisingly appears to be potentiated by the presence of the CS3 operon. The exact component which is instrumental in this potentiation effect is at present unknown.

## 11. Adherence of recombinant Vc/ETEC strains to Caco-2 cells

The test was performed using the human gut cells Caco-2. Caco2-cells were grown at 37°C under 5% $CO_2$ to near confluence in a 24-well plate in MEM medium (GIBCO BRL, Gaithersburg, USA) supplemented with 10% fetal calf serum, thus containing roughly $10^6$ Caco-2 cells per well. The recombinant strains to test, as well as the recipient strain CVD 103-HgR, were grown on CF plates for over 16 h at 37°C. A cell suspension was then made in 0.9% NaCl and the $OD_{600}$ measured. The cells were then diluted in prewarmed MEM medium to 2x $10^6$ CFU/ml. The diluted cells were then further diluted and plated onto BHI plates in duplicate to score the initial viable count (IVC). The medium was removed from the wells containing the Caco-2 cells and replaced by 0.5 ml of the diluted bacterial cells (1:1 multiplicity of infection). Each sample was run in triplicate. The plate was then centrifuged 1'000 rpm for 2 min and incubated at 37°C for 1 hour. At the end of the incubation, 10 μl of each supernatant is appropriately diluted and 100 μl of the dilutions were plated onto BHI plates in duplicate to score the supernatant viable count (SVC). The medium was removed from the wells, which were then carefully washed 3x with 0.5 ml of 0.9 % NaCl. 0.5 ml 0.9% NaCl were then added to each well, the cells were scraped with an Eppendorf tip and the cell suspension added to an Eppendorf tube. The wells were rinsed with 0.5 ml 0.9% NaCl, which was added to the Eppendorf tube. The Eppendorf tubes were subsequently vortexed at maximum speed for a full 2 min. The cell suspensions were then appropriately diluted and the dilutions were plated onto BHI plates in duplicate to score the adherent viable count (AVC). All plates were incubated for 16-20 hours at 37°C and the colonies were scored.

The adherence of the ETEC/cholera strains was determined relative to that of CVD103-HgR by dividing the AVC for the ETEC-cholera ($AVC_{EC}$) by the AVC for CVD103-HgR ($AVC_{CVD}$), thus producing an adherence index (AI). Since we determined in a preliminary experiment that, in our multiplicity of infection range, the number of adherent cells is directly proportional to the inoculum size, the actual ETEC-cholera inoculum ($IVC_{EC}$) was divided by the actual CVD103-HgR inoculum ($IVC_{CVD}$) to produce a inoculum index (II). Finally, the AI value was normalized for the inocula by dividing AI by II, thus producing the normalized adherence index (NAI). An NAI of 1 indicates an adherence identical to that of CVD103-HgR. An NAI above or below 1 indicates higher or lower adherence than CVD103-HgR, respectively. The data shown in table 5 indicate that the CS6 recombinant strain CHCS6-2 and CVD103-HgR may be similarly adherent. In contrast, CHCFA/I appears to be less adherent than CVD103-HgR, while CHCS3 is more adherent by a factor of about 1.7.

Table 5:

| Adherence of pili-producinq ETEC strains | | | | | |
|---|---|---|---|---|---|
| Test pili[a] | Experiment | R[b] | AI[c] | NAI[d] | Average NAI |
| CFA/I | 1 | 0.27 | 0.06 | 0.22 | |
| | 2 | 1.09 | 0.74 | 0.68 | 0.40 |
| | 3 | 0.71 | 0.21 | 0.30 | |
| CS3 | 1 | 0.59 | 1.16 | 1.96 | 1.70 |
| | 2 | 0.25 | 0.36 | 1.44 | |
| CS6 | 1 | 0.22 | 0.19 | 0.84 | |
| | 2 | 1.01 | 1.2 | 1.18 | 1.03 |
| | 3 | 1.24 | 1.35 | 1.08 | |

[a] Strains: CVD103-HgR (reference strain), CFA/I: CHcfal-2 (pM392), CS3: CHCS3-2 (pM392), CS6: CHCS6-2

[b] R: viable count ratio of the pili-producing strain inoculum and the CVD 103-HgR inoculum.

[c] AI: adherence index: viable count ratio of the pili-producing strain adherent cells and the CVD 103-HgR adherent cells

[d] NAI: normalized AI: AI divided by R

## 12. Surface expression: immunofluorescence, electron microscopy.

12.1. Immunofluorescence

To determine the immunofluorescence, the method of Lang et al, 1990 was followed as outlined in Example 10. The test strains were CHcfal-2 (pM392) expressing CFA/I pilin, CHCS3-2 (pM392) expressing CS3, and CHCS6-2 expressing CS6. The primary antibodies were anti-CFA/I, anti-CS3, and anti-CS6 rabbit polyclonal antisera which have been adsorbed against CVD103-HgR and E. coli K12. The parent strain CVD103-HgR served as the negative control for each antiserum.

The results shown in figure 12 indicate that for each antiserum, the negative control CVD103-HgR does not present any fluorescence. In contrast, each of the recombinant strains incubated with the specific antiserum displays a robust fluorescence indicative of surface expression of the pili.

12.2. Electron microscopy

In addition to immunofluorescence, the presence of pili on the surface of the recombinant strains was studied by scanning electron microscopy. The strains tested were CHcfal-2 (pM392) and CHcfal-1 (pM392) expressing CFA/I, CHCS3-2 (pM392) expressing CS3, and CHCS6-1 and CHCS6-2 expressing CS6. The CFA/I positive controls were the ETEC strains H10407, DS7-3, and B7A for the CFA/I, CS3, and CS6 pili, respectively. CVD103-HgR served as a negative control. All strains were grown onto CF plates at 37°C. The cultures were either fixed directly on the agar or adsorbed on glass slides, gold-covered, and poly-L-lysine treated. Following post-fixation treatment with osmium tetraoxide ($OsO_4$) the fixed bacteria were dried by the critical point method or by treatment by hexamethyldisilazane (HMDS). Finally, a 5 nm layer of carbon/platinum was deposed on the samples by electron beam evaporation. All samples were examined using a LEO DSM 982 scanning electron microscope.

Figure 13 shows in panel A the wild-type ETEC strains H10407, expressing CFA/I pili (upper image) and the corresponding recombinant CVD103-HgR/ETEC strain CHcfal-2 (pM392) (lower image), in panel B, the wild-type ETEC strains DS7-3, expressing CS3 pili (upper image) and the corresponding recombinant CVD103-HgR/ETEC strain CHCS3-2 (pM392) (lower image), and in panel C, the wild-type ETEC strain B7A, expressing CS6 pili (upper image) and the corresponding recombinant CVD103-HgR/ETEC strain CHCS6-2 (lower image). In panel D, the V. cholerae host strain CVD 103-HgR is depicted. The results show that each of the recombinant strains expresses surface pili similar to those of the corresponding wild-type ETEC strain. In contrast, CVD103-HgR appears to have a naked surface.

Therefore, we conclude from these experiments that pili are indeed synthesized on the surface of the recombinant CVD103-HgR strain.

### 13. *V. cholerae* **CVD103-HgR characteristics**

It is desirable that the ETEC/cholera vaccine strains conserve the same critical characteristics as the host strain CVD103-HgR. Therefore, the following characteristics were studied: LPS pattern, toxicity, and the production of the B-subunit of cholera toxin (Ctx-B).

To assess the LPS pattern, overnight cultures of the various vaccine strains were grown in LBHySoy at 37°C. CVD103-HgR was used as the control. LPS minipreparations were prepared as described (Hitchcock et al., 1983).

Figure 14 indicates that there is no difference between the CVD103-HgR and the ETEC/cholera vaccine strain LPS Furthermore, the LPS pattern of the wild-type ETEC strains encoding the CFA/I, CS3, and CS6 pili is easily distinguished from that of *V. cholerae.* Therefore, the host strain is thereby identified as *V. cholerae* and the expression of ETEC pilin does not affect the *V. cholerae* LPS pattern.

To assess toxicity, the Y1 adrenal assay was used as described (Sack and Sack, 1975). In this test, the rounding of cells indicating cytotoxicity is qualitatively described. For the production of Ctx-B, the GM1-ELISA assay was used as described previously. (Svennerholm and Holmgren 1978). In this assay, the binding of Ctx-B to GM-1 gangliosides is detected by means of specific anti-Ctx-B antibodies.

Table 6:

| CT-B production and cytotoxicity in recombinant strains | | | |
|---|---|---|---|
| Strain | CT-B (µg /ml)[a] | | Y1 adrenal assay[b] |
| | Test 1 | Test 2 | |
| CH CS6-1 | 9.14 | nd | + |
| CH CS6-2 | 5.92 | nd | + |
| CH cfal-1 pM392 | 1.41 | 17.45 | - |
| CH cfal-2 pM392 | 1.23 | 12.80 | - |
| CH CS3-2 pM392 #1 | 7.89 | 13.70 | - |
| CH CS3-2 pM392 #2 | 2.78 | nd | - |
| CVD 103HgR | 8.77 | 12.49 | + |

[a] The minimum value required for the production of Ctx-B is 2 µg /ml.
[b] - = no rounding, + = 0-25%; ++ = 25-75%; +++ = 75%-100%

The figures in table 6 indicate that the CT-B is equally well produced by CVD103-HgR as by the recombinant strains. In the first GM1-ELISA assay some strains were unexpectedly low. However, these strains showed normal values upon retesting. Likewise, cytotoxicity of the recombinant strains sand CVD103-HgR is identically low.

In conclusion, it appears that the expression of ETEC pilin does not alter any of the critical properties of the CVD103HgR host strain.

### 14. Immunogenicity in mice

Groups of 5 mice were immunized subcutaneously with 100 µl of 0.9% NaCl containing $10^9$ heat-inactivated cells (60 min at 65°C) of the various recombinant strains. The negative control was the vector strain CVD103-HgR and the positive controls were the wild-type ETEC strains. A booster immunization was administered at day 14 and the

**EP 1 543 836 A1**

mice were sacrificed at day 29. The results were analyzed by ELISA using purified pili preparations as the coating antigens. Briefly, 2.5 microgram/ml of the specific pili was coated on a blocked ELISA plate (Nunc MAXISORP, Milian, Switzerland). After washing the wells, 100 microliters of serial dilutions of the serum to test was added and the plate incubated at room temperature for 2 hours. The plate was washed again and 100 microliters of a proxidase-labeled goat anti-mouse IgG antibody was added at a 1:2000 dilution and the plate incubated for 1 h at RT. Finally 100 microliters of a peroxidase substrate solution was added and the plate incubated for 10-15 min at room temperature. The results were then recorded in a plate reader (Spectramax Plus, Bucher Biotec AG, Basel, Switzerland) set at 405 nm. A significant ELISA titer is defined as being equal or superior to twice the reciprocal of the background ELISA titer. Alternatively, mice were immunized with $10^8$ live bacteria at day 1, boosted at day 14 and 28 and sacrificed on day 35.

Table 7:

| Strain | Pili tested | Titer (heat- killed bacteria) | Titer (live bacteria) |
|---|---|---|---|
| CVD103-HgR | CFA/I | <100 | <100 |
| H10407 | CFA/I | 6060 | 33786 |
| CHcfal(pM392) | CFA/I | <100 | ND |
| CHcfal-R1 | CFA/I | ND | 3864 |
| CVD103-HgR | CS3 | <100 | ND |
| DS7-3 | CS3 | 32633 | ND |
| CHCS3 (pM392) | CS3 | 9435 | ND |
| CVD103-HgR | CS6 | <100 | <100 |
| B7A | CS6 | 1598 | 6577 |
| CHCS6-1 | CS6 | <100 | ND |
| CVD103-HgR (pM295) | CS6 | ND | 9156 |

The results given in table 7 showed that a good immune response was obtained from strain CHCS3 (pM392). In contrast no immune response could be obtained from the CFA/I and CS6 recombinant strains. On the assumption that this may have to do with the heat inactivation of the bacteria, new groups of 5 mice were immunized with $10^9$ live bacteria followed by a with $10^8$ live bacteria booster (CFA/I) or by $10^8$ bacteria for both primary and booster immunizations (CS6). The vaccine strain CHcfal-R1 was used for the CFA/I immunization, while strain CVD103-HgR (pM295) was used for the CHCS6-1 immunization. In contrast to immunization with heat-killed bacteria, immunization with live bacteria led to significant immune responses from the CFA/I and CS6 recombinant strains. These experiments show that CVD103-HgR is capable of expressing each of the CFA/I, CS3 and CS6 pili in immunogenic form on its surface.

**Literature**

1. Acheson DW et al 1993, Infect.Immun. 61 3): 1098-1104
2. Altboum Z et al 2001, Infect.Immun. 69 (5): 3150-3158
3. Altboum Z Et al. Infect Immun. 2003 71 (3): 1352-60.
4. Amann E et al. 1988, Gene 69: 301-315
5. Aubel D,et al.. Infect Immun. 1991 Apr;59 (4): 1290-9.
6. Butterton et al 1995, Infect Immun. 63: 2689-2696,
7. Butterton et al 1997, Infect Immun. 65: 2127-2138
8. Caron J et al, 1989, Proc Natl Acad Sci 86 (3): 963-967
9. Caron J et al, 1990 Infect Immun 58:3442-3444
10. Cassels FJ and MK Wolf, 1995. J.Industr.Microbiol., 15: 214-226.
11. Coster TS et al Lancet. 1995 Apr 15;345 (8955): 949-52.
12. Clemens JD et al., 1988, J Infect Dis. 1988 Aug;158 (2): 372-7
13. Cryz S. et al.1993, Infect.Immun. 61:1149-1151.;
14. Darfeuille-Michaud A et al Infect Immun. 1986 May; 52 (2): 468-75.
15. De Haan LA et al, 1991, FEMS Microbiol Lett 67 (3): 341-346

19

**EP 1 543 836 A1**

16. Dietrich G et al. Vaccine. 2003 Jan 30;21 (7-8): 678-83

17. Duthy TG,et al.. J Bacteriol. 1999 181 (18): 5847-51.

18. Favre D. and Viret JF, 2000, Biotechniques 28: 198-204

19. Froehlich BJ et al. Infect Immun. 1995 63 (12): 4849-56.

20. Gaastra W, Svennerholm AM 1996, Trends Microbiol 4 (11): 444-452

21. Gay, CC., 1971 , Ann. N.Y. Acad. Sci. 176: 336-349

22. Germanier and Fürer 1975, J.Infect.Dis. 131: 553-558

23. Giron JA et al. Gene. 1997 Jun 11; 192 (1): 39-43.

24. Grewal HM et al. Infect Immun. 1997 Feb;65 (2): 507-13.

25. Gustafsson B,Holme T.. J Clin Microbiol.;18 (3): 480-5.

26. Heuzenroeder MW et al FEMS Microbiol Lett. 1990 Jan 1;54 (1-3): 55-60.

27. Hitchcock P:J.and T.M. Brown, 1983, J.Bacteriol. 154: 269-277

28. Ketley et al., 1990, Infect Immun, 58: 1481-1484

29. Ketley, JM et al, 1993. FEMS Microbiol Lett 111: 15-22.

30. Kenner JR, et al.O1. J Infect Dis. 1995 Oct; 172 (4): 1126-9.

31. Klipstein F et al. 1981. Infect. Immun. 31:144-150

32. Knutton S, Lloyd DR, McNeish AS Infect Immun. 1987 Jan; 55 (1): 86-92.

33. Lang AB et al1990Hum Antibodies Hybridomas.;1 (2): 96-103.

34. Levine M et al., 1988 Lancet 1988 2: 467-470

35. Levine M et al., Rev. Infect. Dis. 1989. 11 (Suppl 3): 552-567;

36. Levine Mand Hone,1991 In Vaccine and Immunotherapy. Cryz Jr,S.J. (ed.). New York: Pergamon Press Inc, pp. 59-72).

37. Levine M and Kaper, 1993 Vaccine 11, 207-212).

38. Marron MB, Smyth CJ.. Microbiology. 1995 141 :2849-59

39. McConnell MM et al.. J Gen Microbiol. 1989 May; 135 (Pt 5): 1135-44.

40. McConnell MM et al. J Infect Dis. 1990 Feb;161 (2): 343-7.

41. McGhee and Kiyono,1993, Infect.Agents Dis.; 2: 55-73;

42. Mestecky, 1987 J. Clin. Immunol.; 7: 265-276;

43. Morgan R. L., et al., 1978 Infect. Immun. 22: 771-777

44. Nataro JP and Kaper JB., 1988. Clin Microbiol Rev. Jan;11 (1): 142-201.

45. Peltola H et al, 1991 Lancet. ;338 (8778): 1285-9

46. Pichel M, Binsztein N, Viboud G.CS22 Infect Immun. 2000 68: 3280-5.

47. Qadri Fet al 2003 Vaccine 21: 2394-2403

48. Sack DA and Sack RB., 1975 Infect Immun. 1975 Feb;11 (2): 334-6.

49. Sambrook (Sambrook, J., E.F. Fritsch, and T. Maniatis. 1989. In Molecular Cloning: a laboratory manual. 2nd edition. Cold Spring Harbor Laboratory, New York: Cold Spring Harbor Laboratory Press

50. Savelkoul PH et al , 1990, Microb Pathog 8 (2), 91-99

51. Schauder B et al. 1987, Gene 52 : 279-283

52. Schmidt H et al J Clin Microbiol. 1995 Mar; 33 (3): 701-5.

53. Simon RU et al, 1983. Bio/Technology 1: 784-791

54. Svennerholm A.-M. and Holmgren J., 1978. Curr. Microbiol. 1: 19-23.

55. Tacket CO, et al. J Infect Dis. 1995 Sep; 172 (3): 883-6

56. Taniguchi T et al. Infect Immun. 2001 69 (9): 5864-73.

57. Taylor DN et al. Infect Immun. 1999 Apr; 67 (4): 2030-4.

58. Valvatne H et al Infect Immun. 1996 Jul; 64 (7): 2635-42.

59. Viboud GI et al. Infect Immun. 1996 64 (4): 1233-9.

60. Walker, 1994 Vaccine 12: 387-400

61. Wolf MK, 1997 Clin Microbiol Rev. 10 (4): 569-84

62. Wu S. et al 1995 , Infect Immun, 63:4933-4938

**Claims**

1. Recombinant *Vibrio cholerae* strain comprising DNA sequences coding for at least the ETEC pili CFA/I, CS3 and CS6, wherein the DNA sequences are operationally linked to one or more promoters.

2. The strain according to claim 1, further comprising at least one DNA sequence coding for an ETEC pilus selected from the group consisting of CS1, CS2, CS4, CS5, CS7, CS8, CS10, CS11, CS12, CS13, CS14, CS15, CS17, CS18, CS19, CS20, CS21, and CS22.

3. The strain according to claim 1 or 2, wherein the promoter is an ETEC promoter.

4. The strain according to any of claims 1-3, wherein expression of one or more of the ETEC pili is enhanced by at least one ETEC regulator.

5. The strain according to claim 4, wherein said regulator is selected from the group consisting of csfR, rns, cfaD, csvR and aggR.

6. The strain according to claim 5, wherein said regulator is csfR.

7. The strain according to claim 5, wherein said regulator is rns.

8. The strain according to any of claims 1-7, wherein said strain is obtained by introducing the DNA sequences into a recipient strain selected from the group consisting of CVD1 03-HgR, CVD111, CVD112 and Peru-15.

9. The strain according to claim 8, wherein said recipient strain is CVD103-HgR or an equivalent strain.

10. The strain according to any of claims 1-9, wherein the DNA sequences coding for the pili are either present on a plasmid or stably integrated into the chromosome of the recipient strain.

11. The strain according to any of claims 1-10, wherein the DNA sequence coding for the regulator is either present on a plasmid or stably integrated into the chromosome of the recipient strain.

12. The strain according to claim 10 or 11, wherein said DNA sequences are chromosomally integrated into an integration site which is non-essential for inducing a protective immune response by the strain.

13. The strain according to claim 12, wherein the DNA sequences are chromosomally integrated into the hlyA and/or hlyB gene locus.

14. The strain according to claim 13, wherein the locus of integration is hlyB.

15. The strain according to any of claims 1-14, wherein multiple copies of the DNA sequences coding for the pili and/ or regulator are integrated in tandem into a single chromosomal integration site or independently integrated into individual integration sites of the recipient chromosome.

16. The strain according to any of claims 1-15, **characterized in that** expression of said ETEC pili occurs during growth in liquid medium at a temperature between 25°C and 42 °C.

17. The strain according to claim 16, **characterized in that** expression occurs during growth at a temperature of 33°C.

18. The strain according to any of claims 1-17, **characterized in that** the strain has retained the characteristics of the parent strain with respect to LPS and CT subunit B production, and cytotoxicity.

19. The strain according to any of claims 1-18, **characterized in that** the strain shows increased adherence to gut epithelial cells as compared to the recipient strain.

20. Vaccine comprising a strain according to any of the previous claims, optionally in a mixture with a pharmaceutically acceptable excipient and/or buffer.

21. The vaccine according to claim 20, further comprising other bacterial strains and/or toxins.

22. Use of a strain according to any of claims 1-19 for preparing a pharmaceutical composition for immunization against ETEC and/or cholera.

23. The use according to claim 22, wherein the pharmaceutical composition is for oral and/or nasal and/or rectal administration.

24. Method for producing a strain according to any of claims 1-19, comprising the steps of:

(a) introducing the DNA sequences coding for at least the ETEC pili CFA/I, CS3 and CS6 into a strain according to claim 8 and, optionally,
(b) further introducing one or more of the DNA sequence(s) coding for an ETEC regulator according to claim 5.

25. Method for preparing a vaccine composition according to claim 20, comprising formulating the strain in a mixture with a pharmaceutically acceptable excipient and/or buffer.

26. Method for increasing the adherence of a *V. cholerae* strain by introducing at least one DNA sequence coding for an ETEC pilus into said strain and culturing said strain under conditions which allow surface expression of the pilus.

27. The method according to claim 26, **characterized in that** at least one DNA sequence encoding a regulator gene according to claim 5 is further introduced into the strain.

28. Method for enhancing the surface expression of at least one ETEC pilus in a strain according to claim 1 by

(a) introducing at least one DNA sequence encoding a regulator gene into said strain and
(b) culturing said strain under conditions which allow expression of the pilus and the regulator,

wherein the regulator gene is stably integrating into the chromosome of the strain or present on a plasmid.

29. Method for enhancing the surface expression of at least one ETEC pilus other than CS3 in a *V. cholerae* strain by

(a) introducing at least one DNA sequence coding for an ETEC pilus other than CS3 into a strain comprising a CS3 operon and at least one regulator gene stably integrated into the chromosome and
(b) culturing said strain under conditions which allow expression of the pilus and the regulator.

30. The method according to claim 29, **characterized in that** surface expression of CFA/I and/or CS6 is enhanced.

Fig. 1

**Fig. 2**

Fig. 3

Fig. 4

EP 1 543 836 A1

CVD 103-HgR   hlyA::mer-hlyB region

**Fig. 5**

Fig. 6

Fig. 7

1  2  3  4  5  6  7  8  9  10 11 12

**Fig. 8**

CFA/I

CS3

CS6

Fig. 9

CFA/I

1   2   3   4   5   6   7

CS3

1   2   3   4   5   6   7   8   9

CS6

1   2   3   4   5   6   7   8   9

**Fig. 10**

CFA/I

1    2    3    4    5

CS3

1   2   3   4   5   6   7   8   9

Fig. 11

A          B          C

Fig. 12

Fig. 13

A

1 2 3 4 5 6 7 8 9 10

B

1 2 3 4 5 6 7 8 9 10

Fig. 14

**European Patent Office**

# EUROPEAN SEARCH REPORT

Application Number

EP 03 02 9082

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94/01533 A (HARVARD COLLEGE ;VIRUS RES INST (US)) 20 January 1994 (1994-01-20) | 26 | A61K39/108 C12N1/21 |
| Y | * the whole document, particularly page 34, line 25 to page 39, line 24 * | 1-3, 8-10,12, 13,15-26 | |
| | --- | | |
| Y | WO 03/022306 A (DARSLEY MICHAEL JAMES ;STEPHENS JONATHAN CLIVE (GB); TURNER ARTHUR) 20 March 2003 (2003-03-20) | 1-3, 8-10,12, 13,15-26 | |
| A | * the whole document * | | |
| | --- | | |
| Y | WO 00/37106 A (BJARE ULF ;CARLIN NILS (SE); SBL VACCIN AB (SE); ASKELOEF PER (SE)) 29 June 2000 (2000-06-29) * the whole document * | 1-3, 8-10,12, 13,15-26 | |
| | --- | | |
| Y,D | FAVRE D ET AL: "GENE REPLACEMENT IN GRAM-NEGATIVE BACTERIA: THE PMAKSAC VECTORS" BIOTECHNIQUES, EATON PUBLISHING, NATICK, US, vol. 28, no. 2, February 2000 (2000-02), page 198,200,202,204 XP001180140 ISSN: 0736-6205 * the whole document * | 3,12,13 | **TECHNICAL FIELDS SEARCHED (Int.Cl.7)** A61K |
| | --- | | |
| Y | STEFFEN & DUPONT EDS.: "Textbook of travel medicine" 1999 , MARCEL DEKKER , NY XP001180710 Chapter 22.3 "Enteric vaccines: Present an future", by Levine & Svennerholm ISBN: 1550090372.m * page 258, right-hand column, line 5 - page 260, left-hand column, last line * | 1,2 | |
| | --- | | |
| | -/-- | | |

The present search report has been drawn up for all claims

| Place of search | Date of completion of the search | Examiner |
|---|---|---|
| MUNICH | 3 May 2004 | Marinoni, J-C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 1 543 836 A1

**European Patent Office**

## EUROPEAN SEARCH REPORT

Application Number

EP 03 02 9082

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | KETLEY J M ET AL: "Construction of genetically marked Vibrio cholerae 01 vaccine strains" FEMS (FEDERATION OF EUROPEAN MICROBIOLOGICAL SOCIETIES) MICROBIOLOGY, vol. 111, no. 1, 1993, pages 15-21, XP002276310 1993 ISSN: 0378-1097 * the whole document * | 12,13 | |
| A | EP 0 564 689 A (SCHWEIZ SERUM & IMPFINST) 13 October 1993 (1993-10-13) | | |
| A | WO 02/064162 A (CASSELS FREDERICK J ;US GOVERNMENT (US); GLENN GREGORY M (US)) 22 August 2002 (2002-08-22) | | |
| A | WO 03/076643 A (CASSELS FREDERICK J ;WOOD JAMES F (US); U S ARMY MEDICAL RES AND M) 18 September 2003 (2003-09-18) | | TECHNICAL FIELDS SEARCHED (Int.Cl.7) |
| A | CHEN I ET AL: "A RECOMBINANT LIVE ATTENUATED STRAIN OF VIBRIO CHOLERAE INDUCES IMMUNITY AGAINST TETANUS TOXIN AND BORDETELLA PERTUSSIS TRACHEAL COLONIZATION FACTOR" INFECTION AND IMMUNITY, AMERICAN SOCIETY FOR MICROBIOLOGY. WASHINGTON, US, vol. 66, no. 4, April 1998 (1998-04), pages 1648-1653, XP000946419 ISSN: 0019-9567 * abstract * | | |

-/--

The present search report has been drawn up for all claims

| Place of search | Date of completion of the search | Examiner |
|---|---|---|
| MUNICH | 3 May 2004 | Marinoni, J-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

European Patent Office

Application Number

EP 03 02 9082

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DIETRICH G ET AL: "Experience with registered mucosal vaccines" VACCINE, BUTTERWORTH SCIENTIFIC. GUILDFORD, GB, vol. 21, no. 7-8, 30 January 2003 (2003-01-30), pages 678-683, XP004401604 ISSN: 0264-410X --- | | |
| A | ACHESON D W K ET AL: "PROTECTIVE IMMUNITY TO SHIGA-LIKE TOXIN I FOLLOWING ORAL IMMUNIZATION WITH SHIGA-LIKE TOXIN I B-SUBUNIT-PRODUCING VIBRIO CHOLERAE CVD 103-HGR" INFECTION AND IMMUNITY, AMERICAN SOCIETY FOR MICROBIOLOGY. WASHINGTON, US, vol. 64, no. 1, January 1996 (1996-01), pages 355-357, XP001088560 ISSN: 0019-9567 --- | | |
| A | GAASTRA WIM ET AL: "Colonization factors of human enterotoxigenic Escherichia coli (ETEC)" TRENDS IN MICROBIOLOGY, vol. 4, no. 11, 1996, pages 444-452, XP002276311 ISSN: 0966-842X --- | | TECHNICAL FIELDS SEARCHED (Int.Cl.7) |
| A | GALLEGOS MARIA-TRINIDAD ET AL: "AraC/XylS family of transcriptional regulators" MICROBIOLOGY AND MOLECULAR BIOLOGY REVIEWS, vol. 61, no. 4, December 1997 (1997-12), pages 393-410, XP002276312 ISSN: 1092-2172 --- -/-- | | |

The present search report has been drawn up for all claims

| Place of search | Date of completion of the search | Examiner |
|---|---|---|
| MUNICH | 3 May 2004 | Marinoni, J-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 03 02 9082

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DATABASE BIOSIS [Online] BIOSCIENCES INFORMATION SERVICE, PHILADELPHIA, PA, US; 2003 PEI L ET AL: "Development of attenuated Vibrio cholerae vaccine vectors for inducing mucosal immune responses." Database accession no. PREV200300545984 XP002276313 * abstract * & ABSTRACTS OF THE GENERAL MEETING OF THE AMERICAN SOCIETY FOR, vol. 103, 2003, pages E-018, 103rd American Society for Microbiology General Meeting;Washington, DC, USA; May 18-22, 2003, 2003 ISSN: 1060-2011 (ISSN print) --- | | |
| A | DATABASE BIOSIS [Online] BIOSCIENCES INFORMATION SERVICE, PHILADELPHIA, PA, US; November 2003 (2003-11) ZHENG JI-PING ET AL: "Co-expression of CFA/I and CS6 of enterotoxigenic Escherichia coli (ETEC) in Shigella flexneri 2a T32 derivative strain FWL01." Database accession no. PREV200400076585 XP002276314 * abstract * & SHENGWU HUAXUE YU SHENGWU WULI XUEBAO, vol. 35, no. 11, November 2003 (2003-11), pages 1005-1010, ISSN: 0582-9879 (ISSN print) ----- | | TECHNICAL FIELDS SEARCHED (Int.Cl.7) |

The present search report has been drawn up for all claims

| Place of search | Date of completion of the search | Examiner |
|---|---|---|
| MUNICH | 3 May 2004 | Marinoni, J-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 543 836 A1**

European Patent
Office

Application Number

EP 03 02 9082

---

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

41

| European Patent Office | LACK OF UNITY OF INVENTION SHEET B | Application Number EP 03 02 9082 |
|---|---|---|

The Search Division considers that the present European patent application does not comply with the requirements of unity of invention and relates to several inventions or groups of inventions, namely:

1. Claims: 1-25

   Recombinant strain of Vibrio cholerae expressing ETEC pili proteins CFA/I, CS3 and CS6, method for obtaining the same, vaccine comprising said strain, etc...

2. Claims: 26-27

   Method for increasing adherence of Vibrio cholerae by making it express at least one ETEC pilus protein.

3. Claims: 29-30

   Method for enhancing the surface expression of at least one ETEC pilus other than CS3 in a Vibrio cholerae strain.

**ANNEX TO THE EUROPEAN SEARCH REPORT
ON EUROPEAN PATENT APPLICATION NO.**                    EP 03 02 9082

This annex lists the patent family members relating to the patent documents cited in  the above-mentioned European search report.
The members are as contained in the European Patent Office EDP file on
The European Patent Office is in no way liable for these particulars which are merely  given for the purpose of information.

03-05-2004

| Patent document cited in search report | | | Publication date | Patent family member(s) | | | Publication date |
|---|---|---|---|---|---|---|---|
| WO 9401533 | A | | 20-01-1994 | AT | 242319 | T | 15-06-2003 |
| | | | | AU | 4660893 | A | 31-01-1994 |
| | | | | BR | 9306707 | A | 08-12-1998 |
| | | | | CA | 2139655 | A1 | 20-01-1994 |
| | | | | CN | 1083524 | A | 09-03-1994 |
| | | | | DE | 69333028 | D1 | 10-07-2003 |
| | | | | DE | 69333028 | T2 | 22-04-2004 |
| | | | | DK | 672116 | T3 | 06-10-2003 |
| | | | | EP | 0672116 | A1 | 20-09-1995 |
| | | | | ES | 2199944 | T3 | 01-03-2004 |
| | | | | JP | 7508885 | T | 05-10-1995 |
| | | | | MX | 9303992 | A1 | 31-01-1995 |
| | | | | PT | 672116 | T | 31-10-2003 |
| | | | | WO | 9401533 | A1 | 20-01-1994 |
| | | | | US | 5874088 | A | 23-02-1999 |
| | | | | US | 5631010 | A | 20-05-1997 |
| | | | | ZA | 9304736 | A | 24-01-1994 |
| WO 03022306 | A | | 20-03-2003 | WO | 03022306 | A2 | 20-03-2003 |
| | | | | WO | 03022307 | A1 | 20-03-2003 |
| WO 0037106 | A | | 29-06-2000 | SE | 515285 | C2 | 09-07-2001 |
| | | | | AU | 3088900 | A | 12-07-2000 |
| | | | | BR | 9916278 | A | 04-09-2001 |
| | | | | CA | 2352309 | A1 | 29-06-2000 |
| | | | | CN | 1330552 | T | 09-01-2002 |
| | | | | CZ | 20011947 | A3 | 12-12-2001 |
| | | | | EE | 200100309 | A | 15-08-2002 |
| | | | | EP | 1140159 | A1 | 10-10-2001 |
| | | | | HR | 20010433 | A1 | 30-06-2002 |
| | | | | HU | 0104552 | A2 | 29-04-2002 |
| | | | | ID | 29857 | A | 18-10-2001 |
| | | | | JP | 2002532562 | T | 02-10-2002 |
| | | | | NO | 20012889 | A | 12-06-2001 |
| | | | | PL | 348257 | A1 | 20-05-2002 |
| | | | | SE | 9804415 | A | 19-06-2000 |
| | | | | WO | 0037106 | A1 | 29-06-2000 |
| | | | | TR | 200101542 | T2 | 22-10-2001 |
| | | | | ZA | 200104362 | A | 14-01-2002 |
| EP 0564689 | A | | 13-10-1993 | SG | 46987 | A1 | 20-03-1998 |
| | | | | EP | 0564689 | A1 | 13-10-1993 |
| | | | | AT | 155690 | T | 15-08-1997 |
| | | | | DE | 69221120 | D1 | 28-08-1997 |
| | | | | DE | 69221120 | T2 | 26-02-1998 |
| | | | | DE | 564689 | T1 | 27-06-1996 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 03 02 9082

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2004

| Patent document cited in search report | | Publication date | Patent family member(s) | | Publication date |
|---|---|---|---|---|---|
| EP 0564689 | A | | DK | 564689 T3 | 16-02-1998 |
| | | | ES | 2083343 T1 | 16-04-1996 |
| | | | GR | 3024848 T3 | 30-01-1998 |
| WO 02064162 | A | 22-08-2002 | CA | 2437899 A1 | 22-08-2002 |
| | | | EP | 1372708 A2 | 02-01-2004 |
| | | | WO | 02064162 A2 | 22-08-2002 |
| WO 03076643 | A | 18-09-2003 | WO | 03076643 A2 | 18-09-2003 |
| | | | US | 2004005662 A1 | 08-01-2004 |